# EUROPÄISCHE PATENTANMELDUNG

(11) **EP 1 074 613 A1**
(43) Veröffentlichungstag der Anmeldung: **07.02.2001**
(21) Anmeldenummer: 00116161.1
(22) Anmeldetag: 01.08.2000
(51) Int. Cl.: C12N 7/00, C07K 14/155

(54) **Lentivirus aus der Gruppe der Immunschwächeviren der Drillaffen (Mandrillus leucophaeus) und ihre Verwendung**

(30) Priorität: 03.08.1999 DE 19936003
(71) Anmelder: Dade Behring Marburg GmbH, 35041 Marburg (DE)
(72) Erfinder: Gürtler, Lutz Gerhard, Prof. Dr., 17487 Greifswald (DE); Hauser, Hans Peter, Dr., 35041 Marburg (DE); Dongmo Deloko, Yvette Beatrice, 80809 München (DE); Zekeng, Leopold, Dr., Yaoundé (CM); Kaptue, Lazare, Prof., Yaoundé (CM)
(74) Vertreter: Keller, Günter, Dr.

(57) **Zusammenfassung**

Die vorliegende Erfindung betrifft ein Immunschwächevirus der Drillaffen, dessen RNA, die entsprechende cDNA, davon abgeleitete Proteine sowie Fragmente der Nukleinsäuren oder Proteine. Die Erfindung betrifft ebenfalls die diagnostische Verwendung der genannten Nukleinsäuren und Proteine und deren Fragmente sowie ein Diagnostikum.

## Beschreibung

Die vorliegende Erfindung betrifft das Immunschwächevirus SIM27 der Drillaffen, dessen RNA oder ein Teil davon zu der nachfolgend dargestellten Sequenz komplementär ist sowie Varianten dieses Virus. Darüberhinaus sind die virale RNA, die entsprechende cDNA, davon abgeleitete Proteine sowie Fragmente der Nukleinsäuren oder Proteine Gegenstand der vorliegenden Erfindung. Die Erfindung betrifft ebenfalls die diagnostische Verwendung der genannten Nukleinsäuren und Proteine und deren Fragmente sowie ein Diagnostikum enthaltend diese Nukleinsäuren und/oder Proteine und/oder Fragmente davon.

Seit etwa 30 Millionen Jahren entwickeln sich die Primaten, was zu einem hohen Grad der Variabilität der einzelnen Primatenspezies geführt hat. Man unterscheidet die Neuweltaffen (Platyrrhini) von den Altweltaffen (Catarrhini), welche sich ihrerseits in die Menschenähnlichen (Hominoidae) und die Hundskopfartigen (Cercopithecoidea) aufteilen Zusammen mit den Primaten haben sich auch verschiedene Infektionserreger entwickelt, die sich an die individuelle Primatenspezies oder beispielsweise an eine ganze Familie adaptiert haben. Beispiele für Viren sind die affenpathogenen und die humanpathogenen Herpesviren, die zwar noch Individuen einer anderen Primatenspezies infizieren können, aber natürlicherweise nicht von einer auf die andere Primatenspezies übertragen werden Andere Viren infizieren immer noch alle Primaten, wie das Tollwutvirus, das Geibfiebervirus und das Filovirus.

Lentiviren werden unterteilt in die Genera der Spumaviren, der T-Leukaemie/Lymphomaviren und der Immunschwächeviren. Ein Uberblick über die Leukaemie- und Immunschwächeviren der Affen und ihre Pathogenität findet sich in dem Artikel von Hayami (Hayami M et al., Curr. Top. Microbiol. Immunol. 1994; 188: 1-20). Spumaviren scheinen nur in Affen vorzukommen. Nachdem bis heute eine Pathogenität der Spumaviren nicht nachgewiesen worden ist, ist dieses Virus weniger intensiv untersucht als HIV/SIV und HTLV/STLV.

HTLV, die humanen T-Leukaemieviren Typ I und Typ II, sind strukturell sehr ähnlich den STLV, den Simian (Affen)-T-Lymphoma-Viren (Franchini et al., AIDS Res Human Retrovirus 1994; 10: 1047-1060). So ist die Verschiedenheit der Virusspezies, STLV I und II, und der Viren zwischen Menschen (HTLV) und Affen (STLV), ein Zeichen für eine lange individuelle Evolution in den einzelnen Primaten, wenn eine Kreuzübertragung zwischen den verschiedenen Primatenspezies ausgeschlossen werden kann (Franchini et al., AIDS Res Human Retrovirus 1994; 10: 1047-1060). STLV infizierte Affen kommen auf der ganzen Welt vor (Hayami M et al., Curr. Top. Microbiol. Immunol. 1994; 188: 1-20), während SIV-infizierte Affen natürlicherweise nur in Afrika zu finden sind, welches ein Hinweis dafür ist, dass sich sehr wahrscheinlich SIV später entwickelt hat als STLV.

Molekularbiologische Ergebnisse zeigen eindeutig, dass HIV-1 sehr eng mit den Immunschwächeviren des Schimpansen verwandt ist. Letztere Viren werden im weiteren als SIV-1 bezeichnet, während das Virus der Mangaben, SIVsm, als SIV-2 bezeichnet wird. SIV-1 und HIV-1 stammen mit hoher Wahrscheinlichkeit von einem Vorläufervirus ab, ebenso wie SIV-2 und HIV-2 wahrscheinlich einen gemeinsamen Vorläufer haben. Affenhorden können natürlicherweise bis zu 25% mit SIV-2 durchseucht sein, ohne dass Zeichen der Viruspathogenese in den infizierten Tieren erkennbar sind (Chen Z et al., J Virol. 1996; 70: 3617-3627). Bei SIV-2 wurden Infektionen des Menschen nachgewiesen, die sich in ihrer Pathogenese nicht von einer HIV-2-Infektion unterschieden. SIV-2 ist eng mit HIV2 verwandt und besonders in Westafrika südlich der Sahara epidemisch verbreitet, in der gleichen Region, in der die Mangaben leben (Gao FL et al., Nature 1992; 358: 495-499). Die Ergebnisse der Untersuchungen an SIV zeigen, dass neben dem SIV-2 (SIVsm) von Mangaben die Immundefizienzviren der afrikanischen grünen Meerkatze einen weiteren Typ, vielleicht SIV-3, repräsentieren und ferner inzwischen einige weitere Affen-SIV isoliert worden sind, die nicht den erwähnten Gruppen von Viren zugeordnet werden können und die wahrscheinlich den SIV Typ 4 repräsentieren. Dieser SIV-4 Typ wird gebildet von den Viren der Sykes-Affen (Cercopithecus mitis), der Hoest-Affen (Cercopithecus l'hoesti) (Hirsch VM et al. J. Virol. 1999; 73: 1036-1045), der Rotkappen-Mangaben (Cercopithecus torquatus torquatus) (Georges-Courbot MC et al., J. Virol 1998; 72: 600-608), der Mandrill-Affen SIVmnd (Mandrillus sphinx) (Tsujimoto H et al, Nature 1989; 341: 539-541), und der Drillaffen (Mandrillus leucophaeus) (Clewly JP et al., J. Virol. 1998; 72: 10305-10309). Alle bisher isolierten SIV-4 können auf humanen peripheren Blutlymphozyten gezüchtet werden und einige auf der humanen permanenten Zellinie Molt4 clone 8 (Hirsch VM et al. J. Virol. 1999; 73: 1036-1045), was darauf hindeutet, dass auch die Infektion des Menschen mit diesen Viren möglich sein sollte. Der SIV-4 Typ ist vom SIV-2 Typ so verschieden, dass ein SIVmac(SIV2)-spezifischer p25-Antigentest im Überstand von infizierten Zellen produziertes SIVhoest(SIV4) nicht erfassen kann (Hirsch VM et al. J. Virol. 1999; 73: 1036-1045), da die Gag-Region für die Erkennung durch monoklonale Antikörper zu divergent ist. Auch der phylogenetische Vergleich der Nukleinsäuresequenzen der Affenviren zeigt, dass sich das hier beschriebene SIV-4 von SIV-2 und SIV-3 unterscheidet (Korber et al. Human Retroviruses and AIDS 1997. A compilation and analysis of nucleic acid and amino acid sequences. Los Alamos National Laboratory, New Mexico, 1998).

Wie oben beschrieben, ist ein dem SIVcpz ähnliches Virus möglicherweise der Vorläufervirus der menschliche HIV-1-Infektionen verursachenden Viren, worauf die hohe Ähnlichkeit von Viren der Gruppe HIV1-M, -N und -O mit SIV-1 hindeutet.

Es gibt bis heute keine Berichte, dass Menschen mit SIV-4 infiziert wurden. Eine nosokomiale Infektion mit SIV-3 oder SIV-2 ereignete sich durch Kontamination der ekzematösen Haut eines Laborassistenten (Khabbaz RF et al., N. Engl. J. Med. 1994; 330: 172-177). Das SIV replizierte sich für eine gewisse Zeit, die ausreichend war zur Induktion einer starken Antikörperantwort, aber nicht ausreichend war, eine permanente Infektion zu etablieren (Khabbaz RF et al., N. Engl. J. Med. 1994; 330: 172-177). Etwa 3,5 Jahre nach der Serokonversion scheint der Laborassistent frei von der Infektion zu sein (Khabbaz RF et al., N. Engl. J. Med. 1994; 330: 172-177). Ob dieser Weg der Viruselimination der Regelfall ist oder ob auch persistierende Infektionen mit entsprechender Pathogenese aus dem Infektionsereignis resultieren können, ist unbekannt.

Nachdem bisher keine epidemiologischen Studien an Zielgruppen in Zentralafrika durchgeführt wurden, die zeigen können, ob variante Viren wie SIV-4 auch in der menschlichen Population zirkulieren, kann eine Infektion des Menschen nicht bestätigt, aber auch nicht ausgeschlossen werden.

Wie sich am Beispiel des HIV-1 Subtyps O zeigte, waren Antikörpernachweisteste auf Basis von HIV-1 Subtyp M nicht genügend reaktiv, um alle Subtyp O-Infizierten sicher nachweisen zu können (Simon F et al. AIDS 1994; 8: 1628-1629). Die Diagnose einer Infektion mit einem aberranten humanpathogenen SIV-Subtypen könnte wahrscheinlich ebenfalls nicht gestellt werden, da angenommen werden muß, dass die auf HIV-1- und/oder HIV-2-Antigenen beruhenden ELISA-Suchtests negativ sind oder nur gering reaktiv wären, und der Versuch einer Bestätigung über den Immunoblot eine negatives oder vielleicht fragliches Ergebnis erbrächte. Auch über die Nukleinsäuretests könnte die Diagnose wahrscheinlich nicht gestellt werden, da mit den gegenwärtig verfügbaren Tests beispielsweise weder die Nukleinsäure der Viren der Gruppe O noch die von HIV-2 zuverlässig amplifiziert werden kann (Gürtler L et al., 12th World AIDS Conference Geneva Basic Science 1: 121-124).

Die hier beschriebenen Drillaffen (Mandrillus leucophaeus) sind Tiere, die aus der westlichen Region von Kamerun an Nigeria grenzend stammen und dort im Buschland wild leben. Drillaffen sind in der mittel-west-afrikanischen Region weit verbreitet gewesen. Die Tiere werden gejagt und verzehrt, weswegen ist der Bestand in den letzten Jahren laufend zurückgegangen ist. Jungtiere werden teilweise aufgelesen und in der Nähe der Häuser wie Haustiere gehalten. Der hier beschriebene Affe 27 (3 Jahre alt) ist aus freier Wildbahn gefangen und dann über ein Jahr domestiziert worden und hat mit anderen Affen der gleichen oder einer ähnlichen Spezies keinen Kontakt gehabt.

Wie in Beispiel 2 beschrieben, wurde das aus dem Affen 27 stammende Virus in menschlichen PBLs vermehrt. Aus den infizierten Zellen wurde genomische DNA und damit auch integrierte provirale DNA des SIV isoliert. Die Entschlüsselung der Sequenz des Gesamtgenoms des SIV ist in Beispiel 3 beschrieben Für die Vervielfältigung der viralen DNA wurde die Methode der PCR (polymerase chain reaction) eingesetzt. Die zur Durchführung des Verfahrens benötigten Komponenten können käuflich erworben werden.

Mit diesem Verfahren ist es möglich, DNA-Sequenzen zu amplifizieren, wenn DNA-Bereiche der zu amplifizierenden Sequenz bekannt sind, oder bekannten Abschnitten genügend ähnlich sind Es müssen dann kurze komplementäre DNA-Fragmente (Oligonucleotide = Primer) synthetisiert werden, die sich an einen kurzen Bereich der zu amplifizierenden Nukleinsauresequenz anlagern. Für die Testdurchführung werden Nukleinsäuren mit den Primern in eitler Reaktionsmischung zusammengebracht, die zusätzlich eine Polymerase und Nukleotidtriphosphate enthält. Die Polymerisation (DNA-Synthese) wird für eine bestimmte Zeit durchgeführt, dann werden die Nukleinsäurestränge durch Enwärmen getrennt. Nach Abkühlen läuft die Polymerisation erneut an.

Die Sequenzierung der amplifizierten Genomabschnitte erfolgte nach der Sanger Methode. Wie in Beispiel 4 beschrieben, wurde das Genom von SIM27 phylogenetischen Vergleichen unterzogen, die zeigten, daß es sich um ein stark divergentes neues Immundefizienzvirus der Affen handelt.

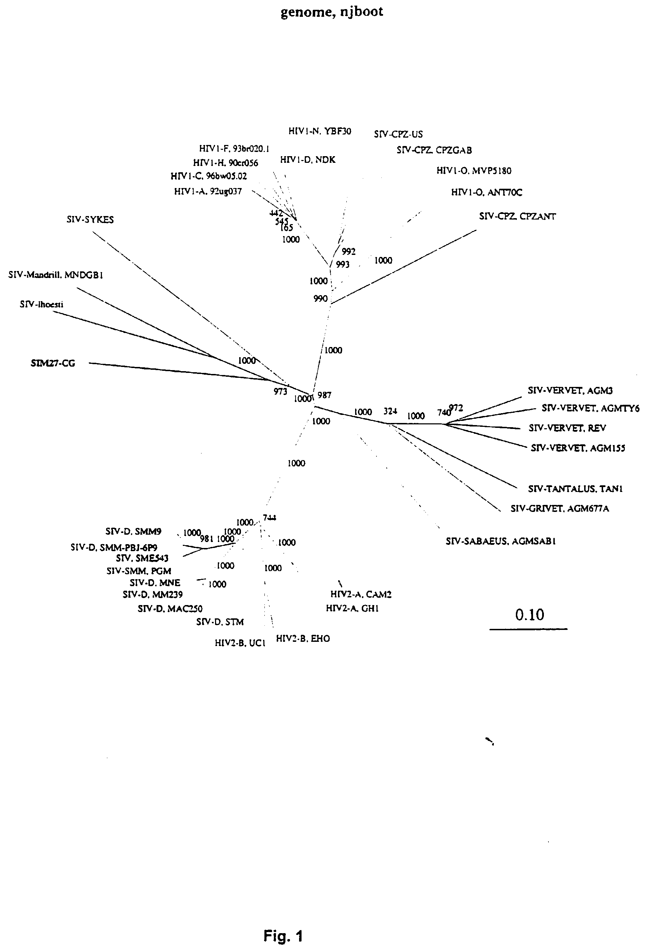

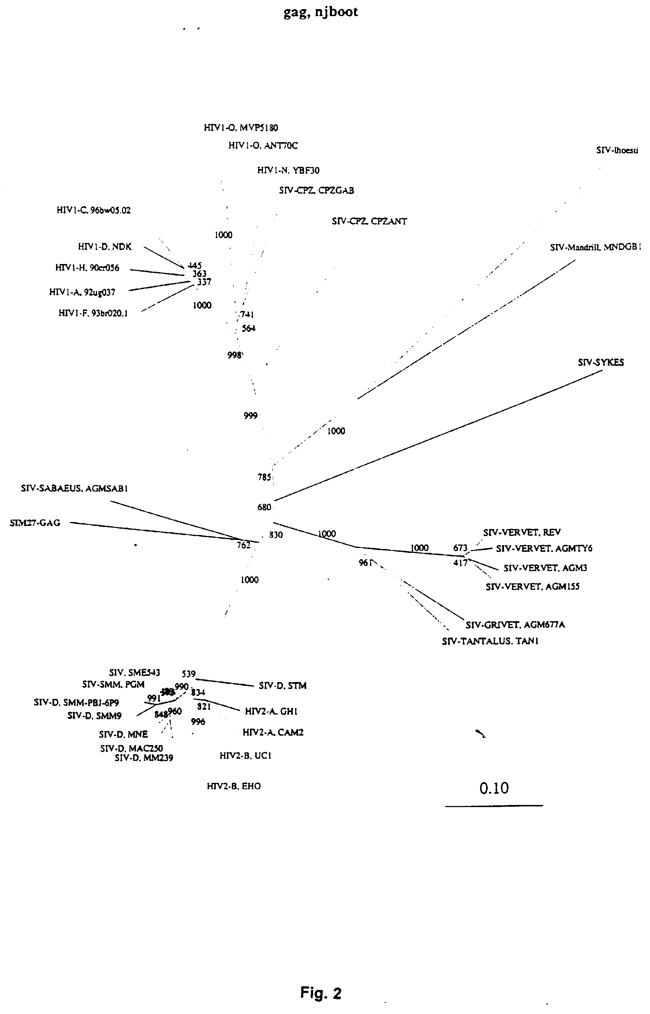

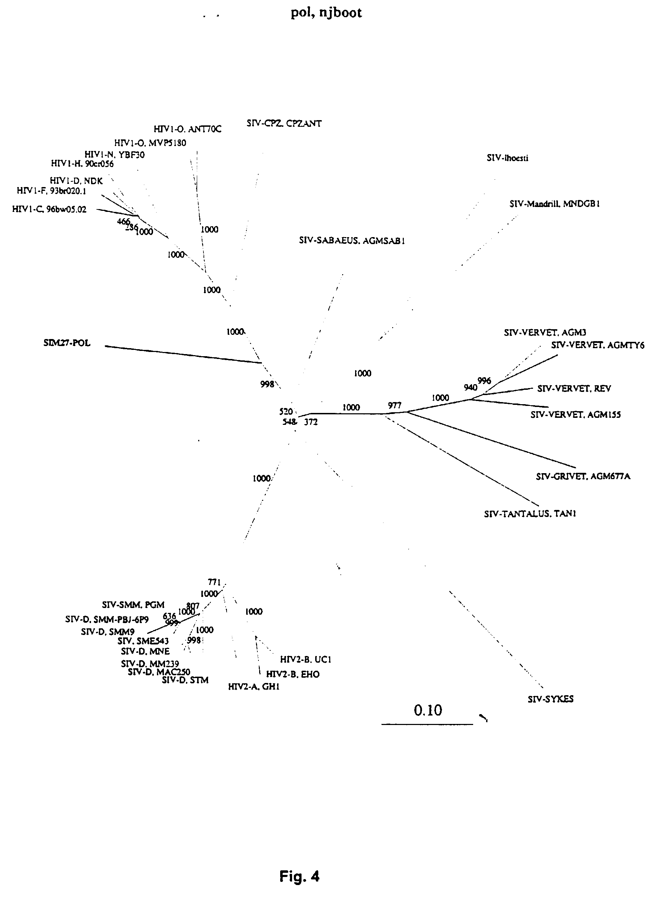

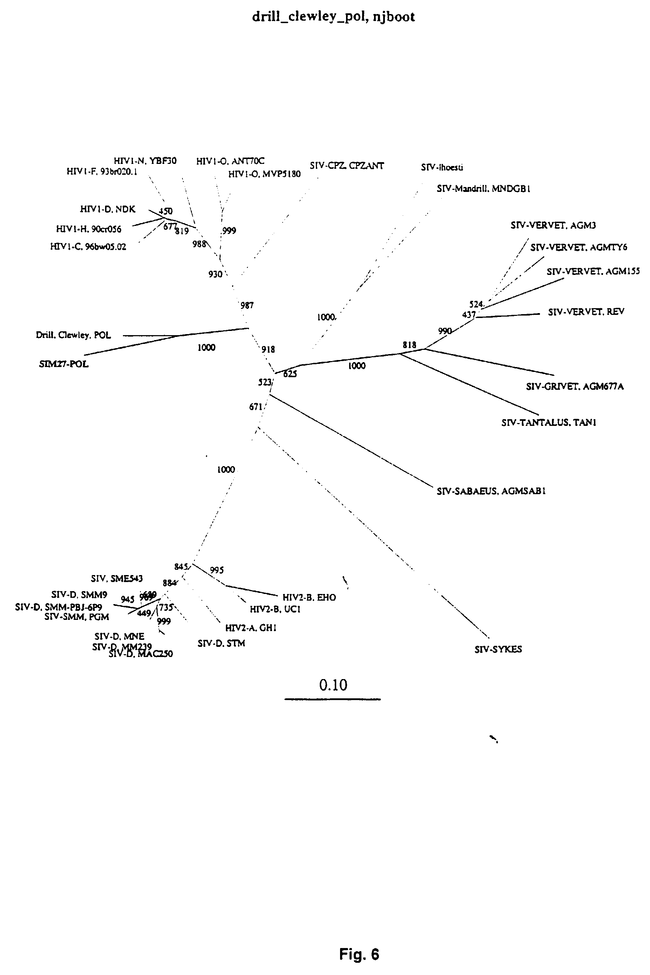

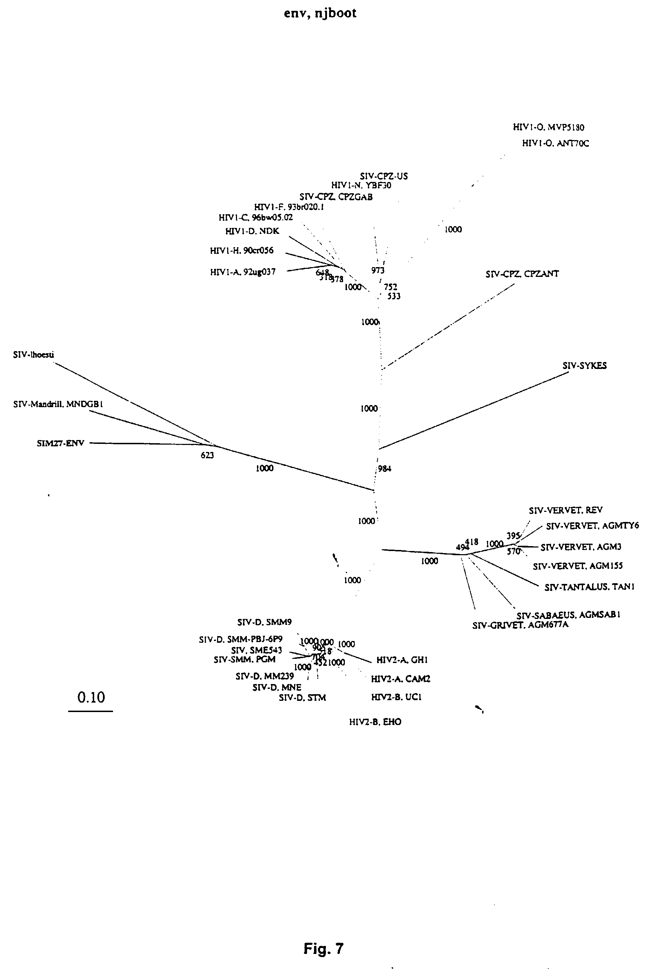

Gegenstand vorliegender Erfindung sind daher:
1.) Immundefizienzviren, die bei einer phylogenetischen Untersuchung ihres Gesamtgenoms auf Nukleinsäureebene, wie sie in Beispiel 4 beschrieben ist, als Seitenast nach der Verzweigung von SIM27 vom SIM27 Seitenast abzweigen (Siehe **Fig.**1).
2.) GAG-Proteine und Fragmente davon, die bei einer phylogenetischen Untersuchung ihrer Gesamtsequenz auf Aminosäureebene wie sie in Beispiel 4 beschrieben ist, als Seitenast nach der Verzweigung von SIM27 vom SIM27 Seitenast abzweigen (Siehe **Fig.**2).
3.) Pol-Proteine und Fragmente davon, die bei einer phylogenetischen Untersuchung ihrer Gesamtsequenz auf Aminosäureebene wie sie in Beispiel 4 beschrieben ist, als Seitenast nach der Verzweigung von SIM27 vom SIM27 Seitenast abzweigen (Siehe **Fig.**4), bzw. Pol-Proteinfragment oder Subfragmente davon, die im Bereich der von Clewley (Clewley JP et al., J.Virol. 1998; 72: 10305-10309) veröffentlichten Sequenz unter Einbeziehung dieser Aminosäuresequenz wie in Beispiel 4 beschrieben untersucht wurden, als Seitenast nach der Verzweigung von SIM27 vom SIM27 Seitenast abzweigen (Siehe **Fig.**6).
4.) Env-Proteine und Fragmente davon, die bei einer phylogenetischen Untersuchung ihrer Gesamtsequenz auf Aminosäureebene wie sie in Beispiel 4 beschrieben ist, als Seitenast nach der Verzweigung von SIM27 vom SIM27 Seitenast abzweigen (Siehe **Fig.**7).

Von besonderem Interesse ist ferner die Betrachtung der stark immunogenen Cysteinloop-Region im Env-Gen, welche daher von besonderer diagnostischer Bedeutung ist. Die Cysteinloop-Regionen verschiedener Immundefizienzviren sind in Tab. 1 dargestellt.

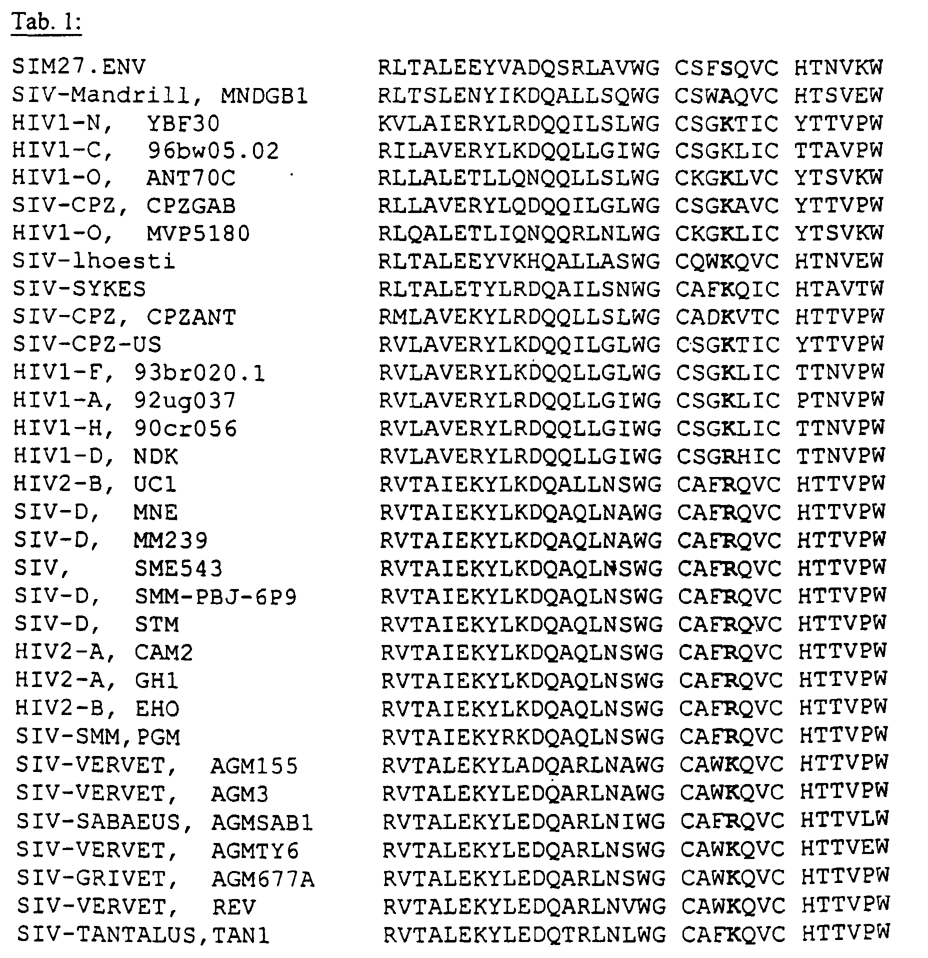

Wie klar ersichtlich ist, kommt an Position 3 des Cysteinloops (C12345C) bei nahezu allen Vertretern von Immundefizienzviren entweder Lysin oder Arginin vor. Die einzige Ausnahme wurde bisher bei dem Immundefizienzvirus MNDGB1 gefunden, welches ebenfalls aus einem Drillaffen (Mandrillus spinx) isoliert wurde. Es ist mit großer Wahrscheinlichkeit davon auszugehen, daß gegen dieses veränderte Epitop gebildete Antikörper, nicht oder mit deutlich verringerter Effizienz von bisher bekannten diagnostischen Testen erkannt werden, die auf Basis der üblichen Arginin oder Lysin enthaltenden Antigene beruhen.

Ebenfalls Gegenstand dieser Erfindung sind daher Antigene, bei denen innerhalb des Cysteinloop-Bereichs an Position 3 Arginin und/oder Lysin durch eine beliebige Aminosäure, besonders bevorzugt eine polare Aminosäure wie Serin oder eine Aminosäure mit einer aliphatischen Seitenkette wie Alanin ersetzt wurden.

Die vorliegende Erfindung ist darüberhinaus in den Beispielen sowie in den Patentansprüchen beschrieben, wobei die Beispiele der Präzisierung dienen und daraus keine Einschränkung der vorliegenden Erfindung abgeleitet werden darf.

### Beispiel 1:

### Identifizierung der SIM27-Infektion in Drillaffen

Im Zuge einer Studie wurden Drillaffen in den Dörfern des ländlichen Kamerun, in denen sie gehalten wurden, EDTA- Blut abgenommen und dieses in verschiedenen HIV-Tests analysiert. Bei Testung des Serums des Affen SIM27 auf Antikörper war ein kompetitiver ELISA für HIV-1 negativ, ein HIV-1, -2 und -O erkennender ELISA der Firma Dade Behring (Enzygnost HIV-1/2 plus) war ebenfalls negativ, wobei die Extinktion nahe dem Schwellenwert lag. In der gleichzeitig durchgeführten Analyse des HIV-1 Westernblots (Virus MVP899-87) waren keine virusspezifischen Banden zu sehen, im HIV-2 blot (Virus MVP11971-87) die Banden gp36 stark, p55 und p68, und im HIV-1 Gruppe O blot (Virus MVP5180-91) die Banden p24 und p55. Das gp 36 ist das Transmembranprotein von HIV-2, die Banden p55 und p68 entsprechen der reversen Transcriptase (p55) plus der RNaseH (p68) von HIV-2, und p24 ist das innere Kernprotein von HIV-1 Gruppe O Viren und p55 das Vorläuferprotein von gag und damit auch p24. Aufgetragen wurden 20 ml Plasma der Tiere, um den Westernblot zu entwickeln. Das Virus MVP-11971-87 ist nach der Analyse der Nukleinsäuresequenz ein Vertreter der Gruppe HIV-2A, das Virus MVP-899 ein Vertreter von HIV-1B.

Somit zeichnet sich die SIV-Infektion der Affen mit dem Drillvirus aus:
* durch Negativität in normalen Screening-ELISAs für HIV-Antikörper,
* durch serologische Kreuzreaktion im env- und pol-Bereich mit dem HIV-2 Transmembran-Glykoprotein und der reversen Transcriptase im Western Blot,
* durch serologische Kreuzreaktion im gag-Bereich mit dem inneren Kernprotein von HIV-1 Gruppe O und fehlender Kreuzreaktion mit den Kernproteinen der Gruppe M (HIV-1B) im Western Blot.

### Beispiel 2:

### Isolierung des SIM27 Virus

Aus je 5 ml EDTA-Blut der Affen wurde die Lymphozyten-Fraktion über Ficoll-Gradienten Zentrifugation isoliert. Die Lymphozyten wurden mit PHA (Phytohämagglutinin, 5mg/ml) und PMA (Myristyl-phorbolester, 10ng/ml) stimuliert, nach 3 Tagen beide Zusätze ausgewaschen und die Kultur in Gegenwart von RPMI-1640, wie üblich, mit Interleukin-2 Zusatz fortgesetzt. Die PMA Stimulation wurde von Kubo et al. beschrieben (Kubo M et al., J Virol 1997; 71: 7560-7566).

Die Kulturbedingungen waren ähnlich denen, die von Tamalet et al. beschrieben wurden (Tamalet, C. et al., AIDS 1994; 8: 1083-1088). Nach einer Woche in Kultur wurden zu den Affen-Lymphozyten menschliche PHA-stimulierte und nicht-stimulierte Blutlymphozyten (PBL) zugegeben und die Zugabe wöchentlich einmal wiederholt, bis nach ca. 3 Wochen über einen kommerziell erhältlichen p24-Antigentest (Abbott, Wiesbaden) die beginnende SIV-Produktion nachgewiesen werden konnte.

Das Virus wurde dann aus dem Überstand der Zellen auf menschlichen Lymphozyten subkultiviert. Alle Versuche, das SIM27 auf permanente Kulturzellen wie HUT-78 oder Jurkat zu übertragen, sind bisher fehlgeschlagen. Über monatliche Subkultivierungen konnte SIM27 seither während 9 Monaten auf PBL in Kultur gehalten werden

### Beispiel 3:

### DNA-Isolierung, Amplifizierung und strukturelle Charakterisierung von Genomabschnitten des HIV-Isolates SIM27

Genomische DNA aus SIM27-infizierten Blutlymphozyten wurde nach Standardmethoden isoliert (Current Protocols in Molecular Biology, Wiley Interscience, 1994).

Das Gesamtgenom wurde ausschließlich über PCR (Polymerase Chain Reaction) amplifiziert. Sämtlich PCRs wurden mittels 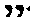 Hot Start" begonnen: nach Zugabe sämtlicher Komponenten der PCR, außer der Polymerase, wurde diese erst nach Erhitzung der Probe auf 94°C zugegeben, was die Extension unspezifisch bindender Primer stark reduziert.

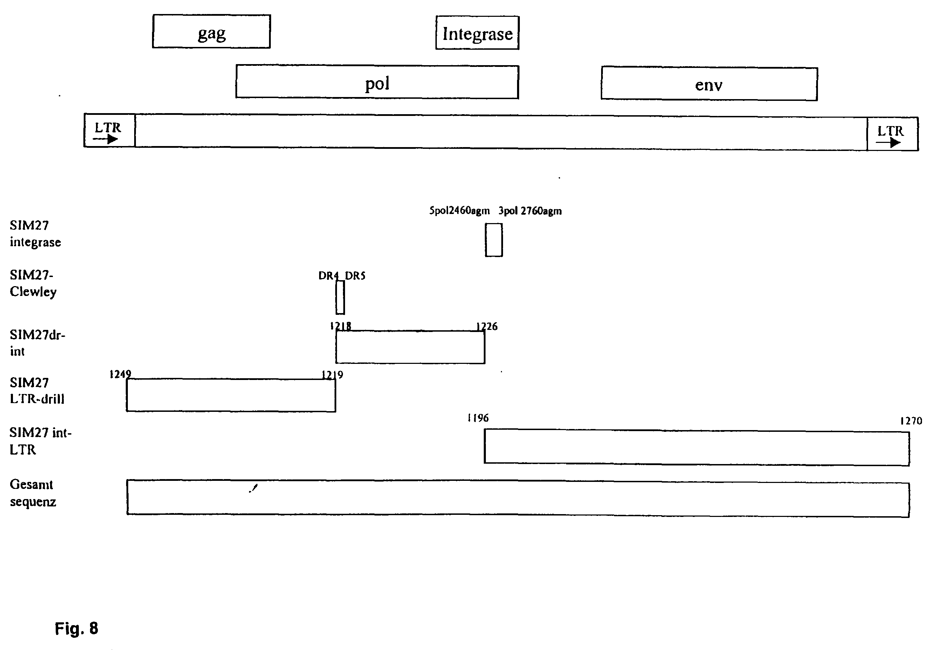

Eine Übersicht der einzelnen Stufen der Entschlüsselung des Genoms ist in Fig. 8 dargestellt.

Zur Charakterisierung von Genombereichen des Isolates SIM27 wurden PCR-Experimente mit Primerpaaren aus dem Bereich der Integrase im pol-Gen durchgeführt. Die PCR (Saiki et al., Science 239: 487-491, 1988) wurde wie folgt modifiziert:

Für die erste Amplifikation HIV-spezifischer DNA Bereiche wurden 5 µl (200 µg/ml) genomische DNA aus SIM27-infizierten Blutlymphozyten in einem 50 µl-Reaktionsansatz (0,25 mM dNTP, je 1 µM Primer, 10 mM Tris HCl, pH 8.3, 50 mM KCl, 1.5 mM MgCl₂, 0,001 % Gelatine, 2,5 units Platinum-Taq-DNA-Polymerase (Gibco)) pipettiert und nach folgendem Temperaturprogramm amplifiziert:
1) Initiale Denaturierung: 3 min. 95°C,
2) Amplifikation: 30 sec. 94°C, 30 sec. 49°C, 30 sec. 68°C (30 Cyclen).

Die für die PCR verwendeten Primer wiesen folgende Sequenz auf:

### (Seq. ID No.1 und 2)

5'                                    3'
5pol2380agm GCC ATG TGT CCA AAA TGT CA
3pol2930agm CTT CTC TGT AGT AGA CTC TA

5 µl des Amplifikates wurden als Template für eine zweite 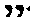 nested" PCR mit den folgenden Primern und demselben Temperaturprofil eingesetzt:

### (Seq. ID No.3 und 4)

5pol2460agm TAG TAG CAG TCC MYR KWG
(M=A/C, Y=C/T, R=A/G, K=G/T, W=A/T)
3pol2760agm TCT CTA ATT TGT CCT ATG AT

Das so erhaltene Amplifikat wurde ohne Klonierung direkt sequenziert.

Die gefundene Sequenz ist in Tab. 2 dargestellt

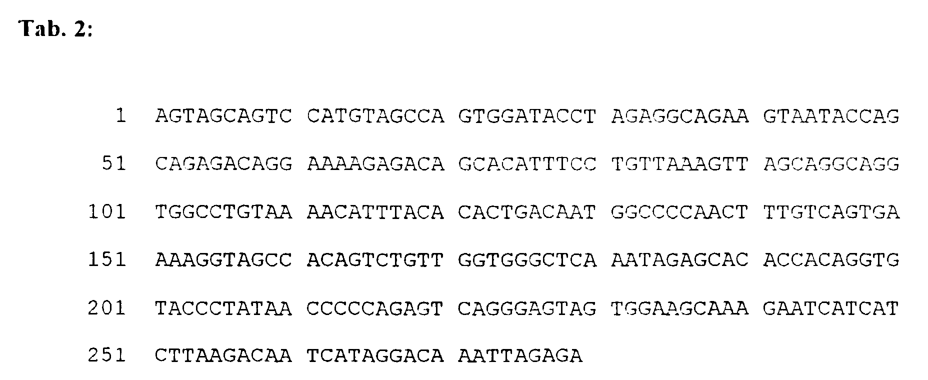

Auf Basis der Veröffentlichung von Clewley (Clewley JP et al., J. Virol. 1998; 72: 10305-10309) wurde ein weiteres Amplifikat im 5' Bereich des pol-Gens erhalten. Verwendet wurden die von Clewley beschriebenen Primer DR1, DR2 sowie für die 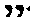 nested" PCR DR4 und DR5, ebenso wie die in dieser Veröffentlichung beschriebenen Temperaturzyklen. Als Polymerase wurden DNA-Taq-Polymerase (Perkin Elmer) und die oben beschriebenen Puffer verwendet.

Hierbei wurde die Sequenz gemäß Tab. 3 erhalten:

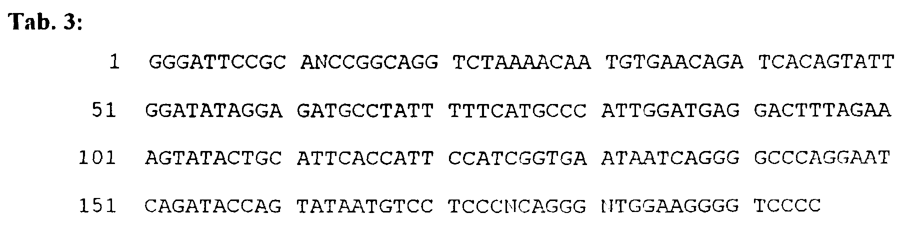

In einer nächsten Amplifikation wurde der zwischen den bereits erhaltenen Amplifikaten liegende Bereich von SIM27 amplifiziert. Hierbei wurden nachfolgend genannte Primer verwendet.

Für die erste PCR:

### (Seq. ID No.5 und 6)

1216 ATG CCC ATT GGA TGA GGA C
1197 GAC TGT GGC TAC CTT TTC ACT

Für die 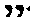 nested" PCR:

### (Seq. ID No.7 und 8)

1218 CAT CGG TGA ATA ATC AGG
1226 GGT ATT ACT TCT GCC TCT A

Verwendet wurde die Platinum-Taq-DNA-Polymerase (Gibco) nach folgendem Temperaturprogramm:
1) Initiale Denaturierung: 2 min. 95°C,
2) Amplifikation: 30 sec. 95°C, 30 sec. 55°C, 150 sec. 68°C (30 Cyclen).

Dabei wurde die Sequenz gemäß Tab. 4 erhalten.

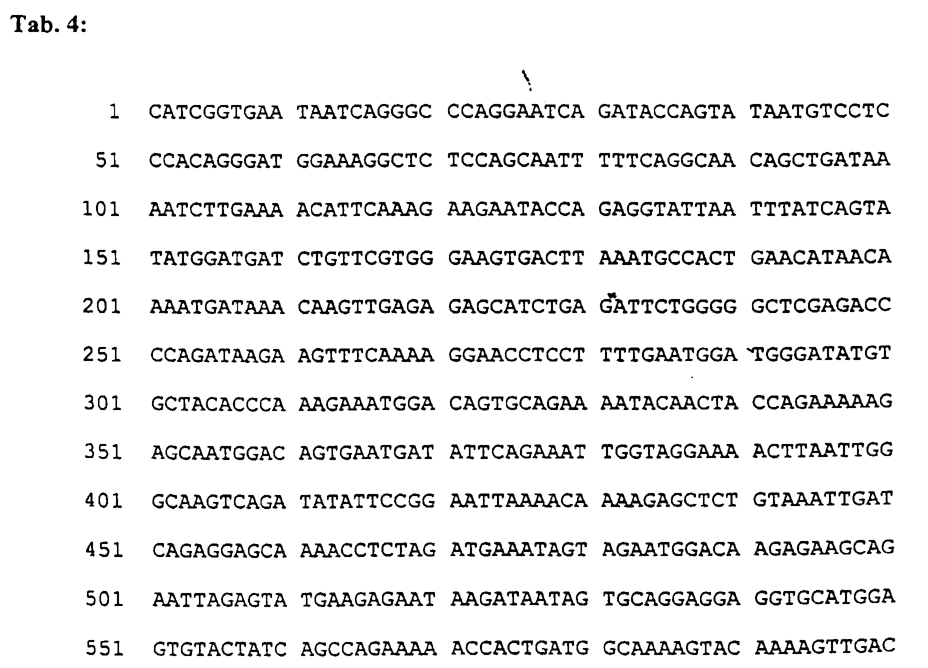

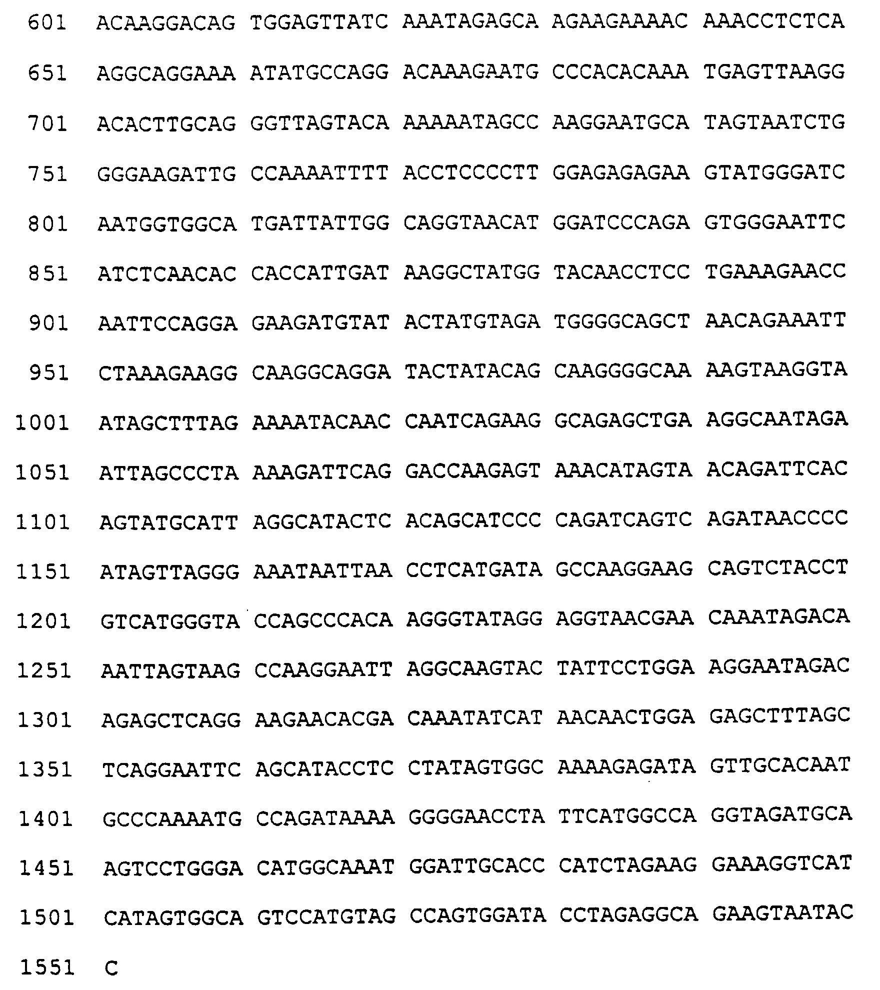

Der Bereich der Gesamtsequenz vom 5'-LTR-Bereich des Genoms bis zum pol-Gen wurde mit folgenden Primerpaaren amplifiziert:

### 1. PCR:

### (Seq. ID No.9 und 10)

1248 CTC AAT AAA GCT TGC CTT GA
1217 GTC CTC ATC CAA TGG GCA T

2. 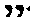 Nested" PCR:

### (Seq. ID No.11 und 12)

1249 TRD CTA GAG ATC CCT CAG A (R=A/G, D=G/A/T)
1219 CCA ATA CTG TGA TCT GTT CAC

Verwendet wurde jeweils die Platinum-Taq-DNA-Polymerase (Gibco) nach folgendem Temperaturprogramm:
1. Initiale Denaturierung: 2 min. 95°C,
2. Amplifikation: 30 sec. 95°C, 30 sec. 50°C, 180 sec. 68°C (30 Cyclen). Zusätzlich zu den oben angegebenen Puffern wurde 1xEnhancer (Gibco) verwendet.

Dabei wurde die Sequenz gemäß Tab. 5 erhalten:

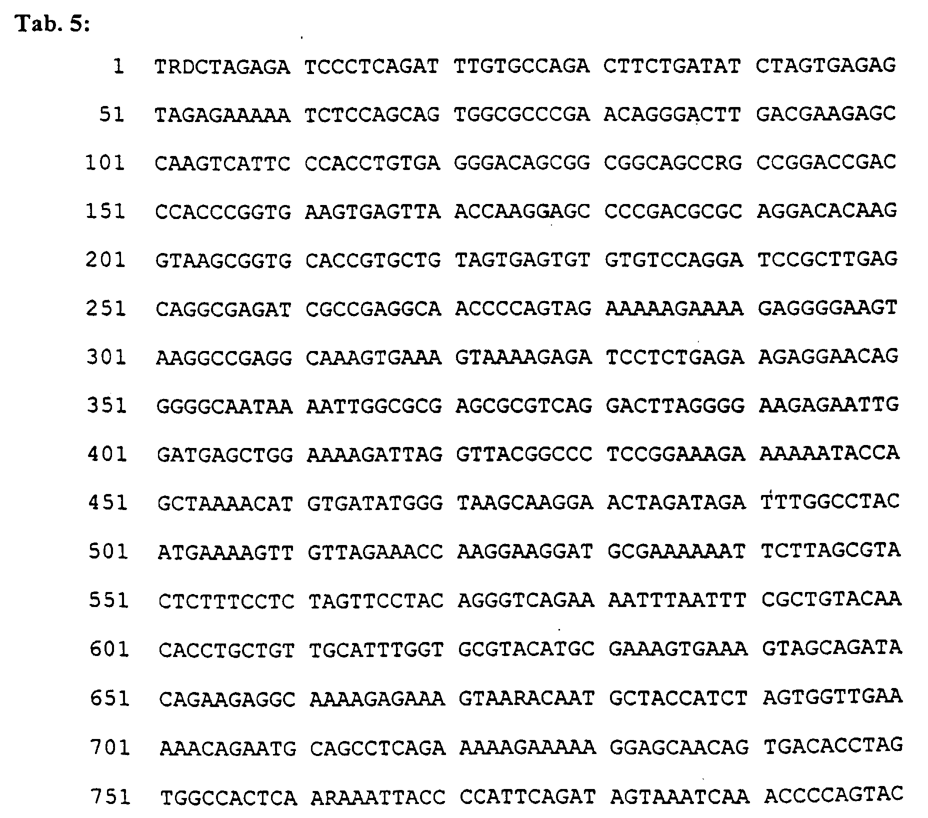

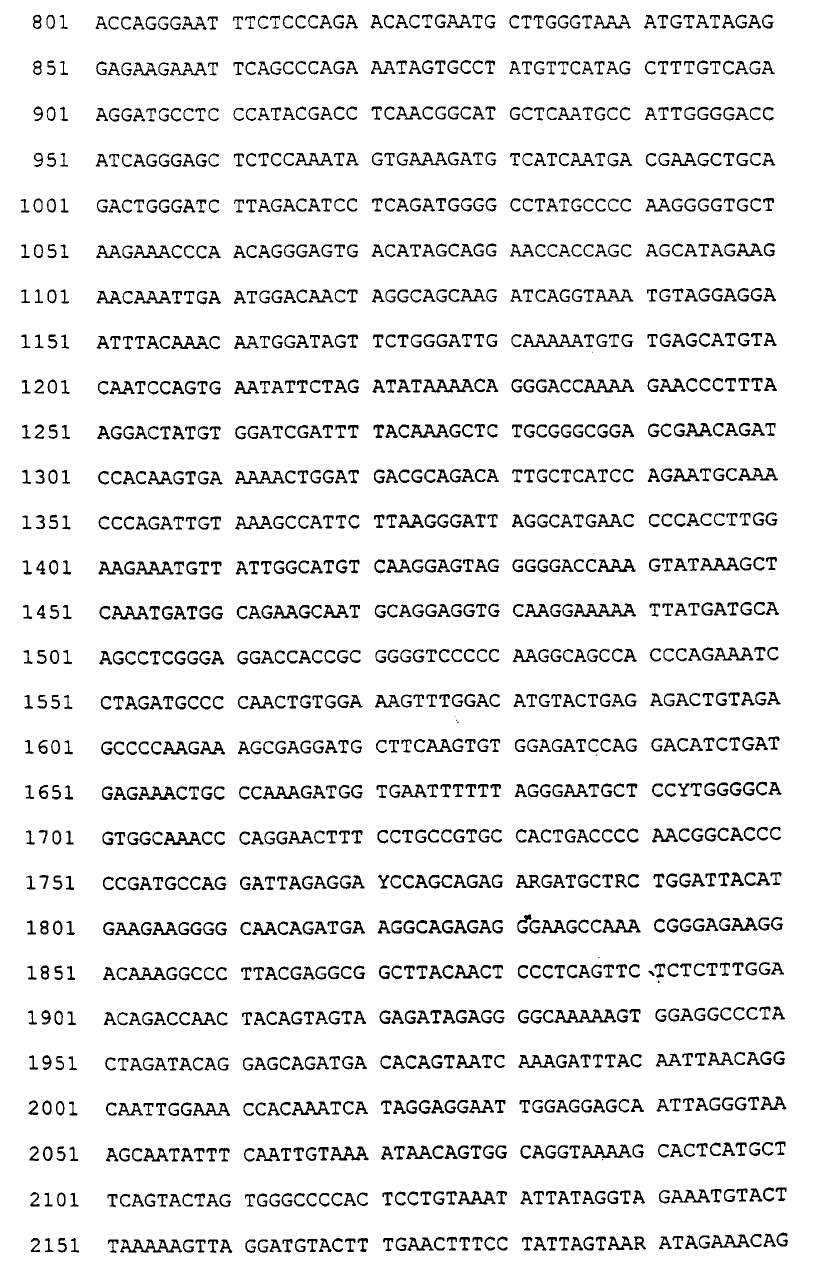

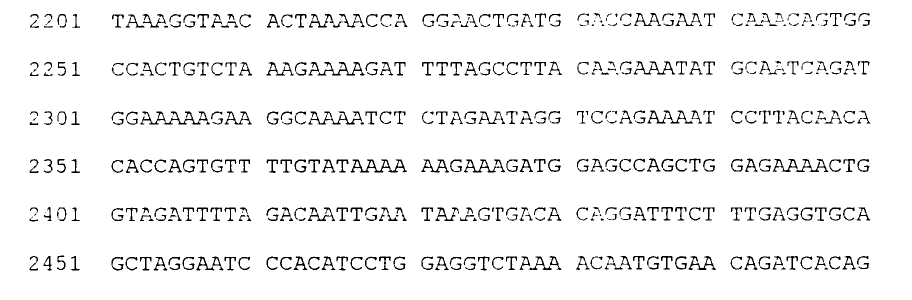

Der noch fehlende Bereich der Gesamtsequenz von der Integrase bis zum 3'-LTR wurde mit folgenden Primerpaaren amplifiziert, wobei der Primer 1270 aufgrund der Sequenz des 5'LTR Bereichs (vorheriges Amplifikat) entworfen wurde:

### 1.PCR:

### (Seq. ID No.13 und 14)

1246 CCT ATT CAT GGC CAG GTA
1270 GAT TTT TCT CTA CTC TCA CTA

2. 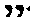 Nested" PCR:

### (Seq. ID No.15 und 16)

1196 AGT GAA AAG GTA GCC ACA GTC
1270 GAT TTT TCT CTA CTC TCA CTA

Verwendet wurde jeweils die Platinum-Taq-DNA-Polymerase (Gibco) nach folgendem Temperaturprogramm:
1. Initiale Denaturierung: 2 min. 95°C,
2. Amplifikation: 30 sec. 95°C, 30 sec. (47°C 1.PCR; 51°C 2.PCR), 360 sec. 68°C (30 Cyclen). Zusätzlich zu den oben angegebenen Puffern wurde 1xEnhancer (Gibco) verwendet.

Dabei wurde die Sequenz gemäß Tab. 6 erhalten:

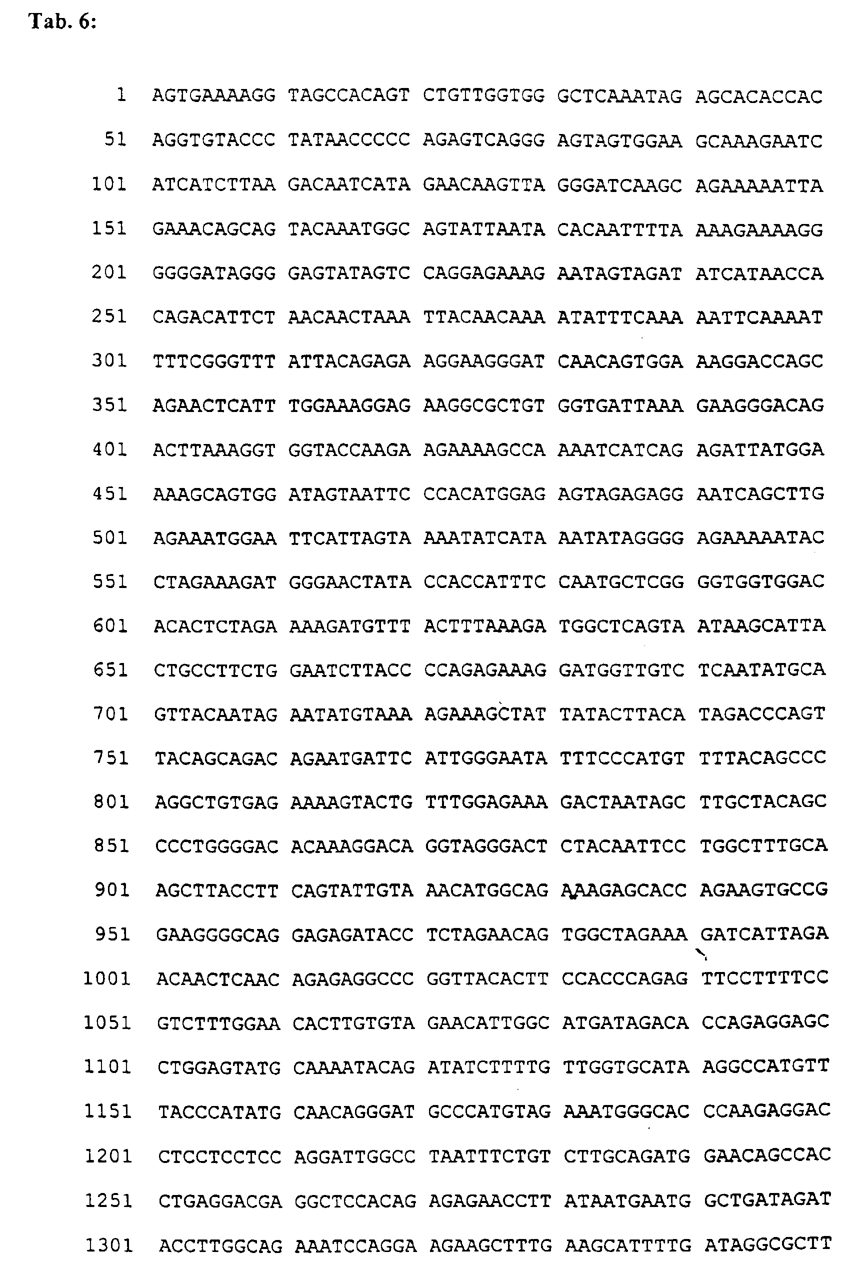

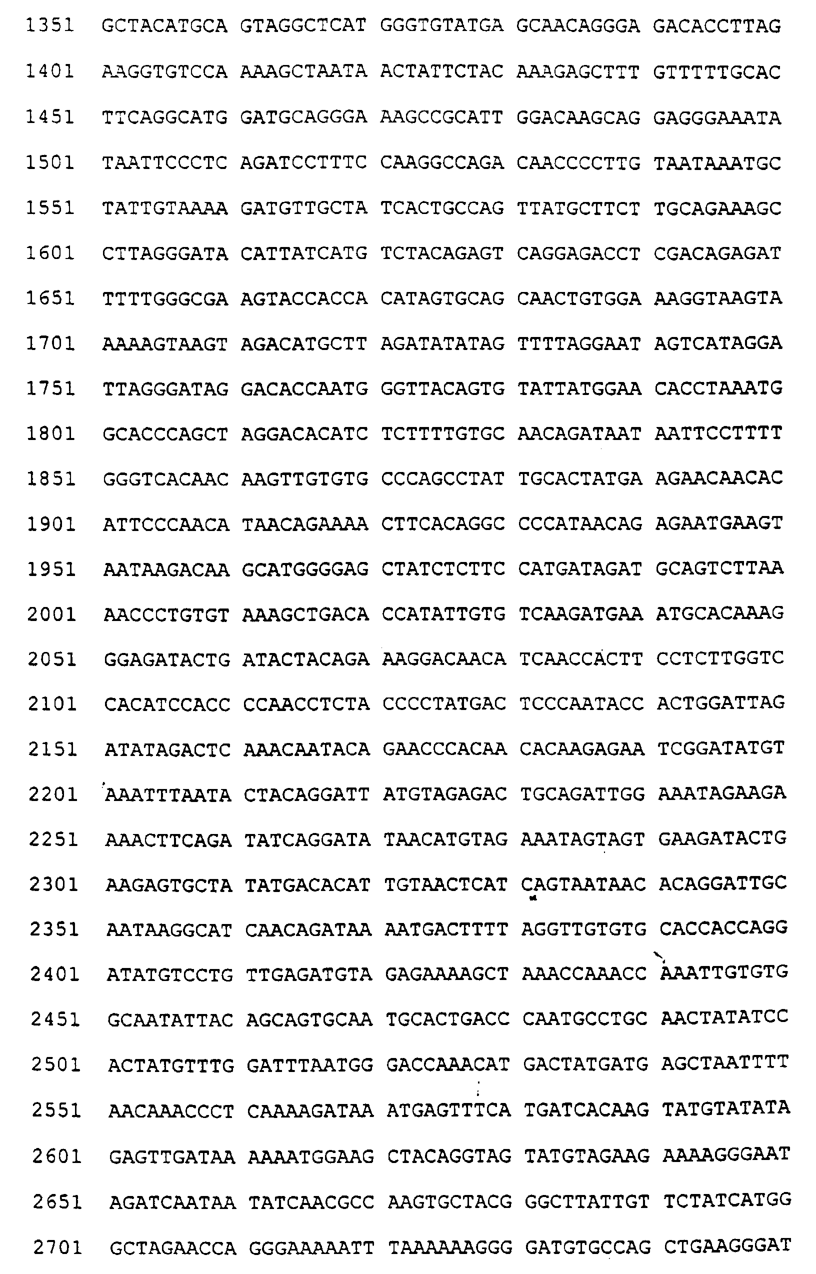

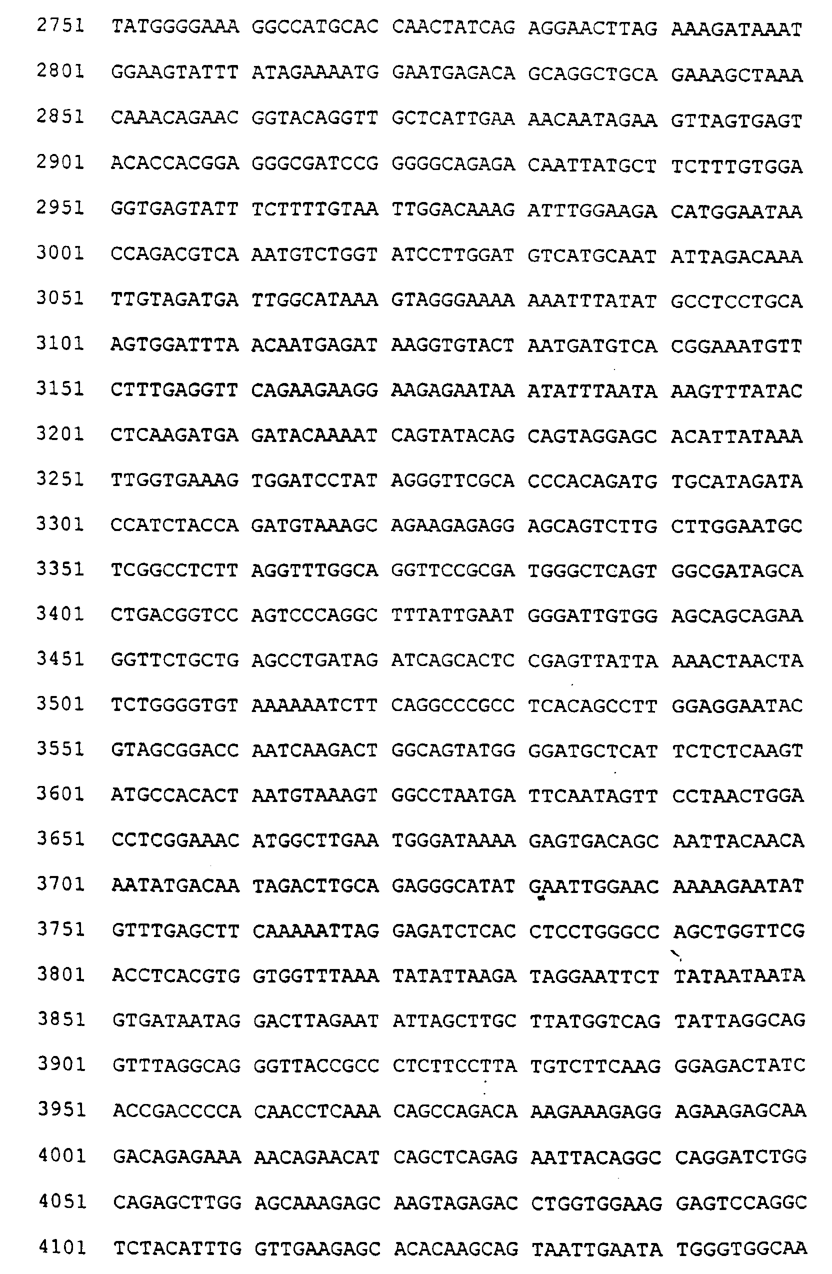

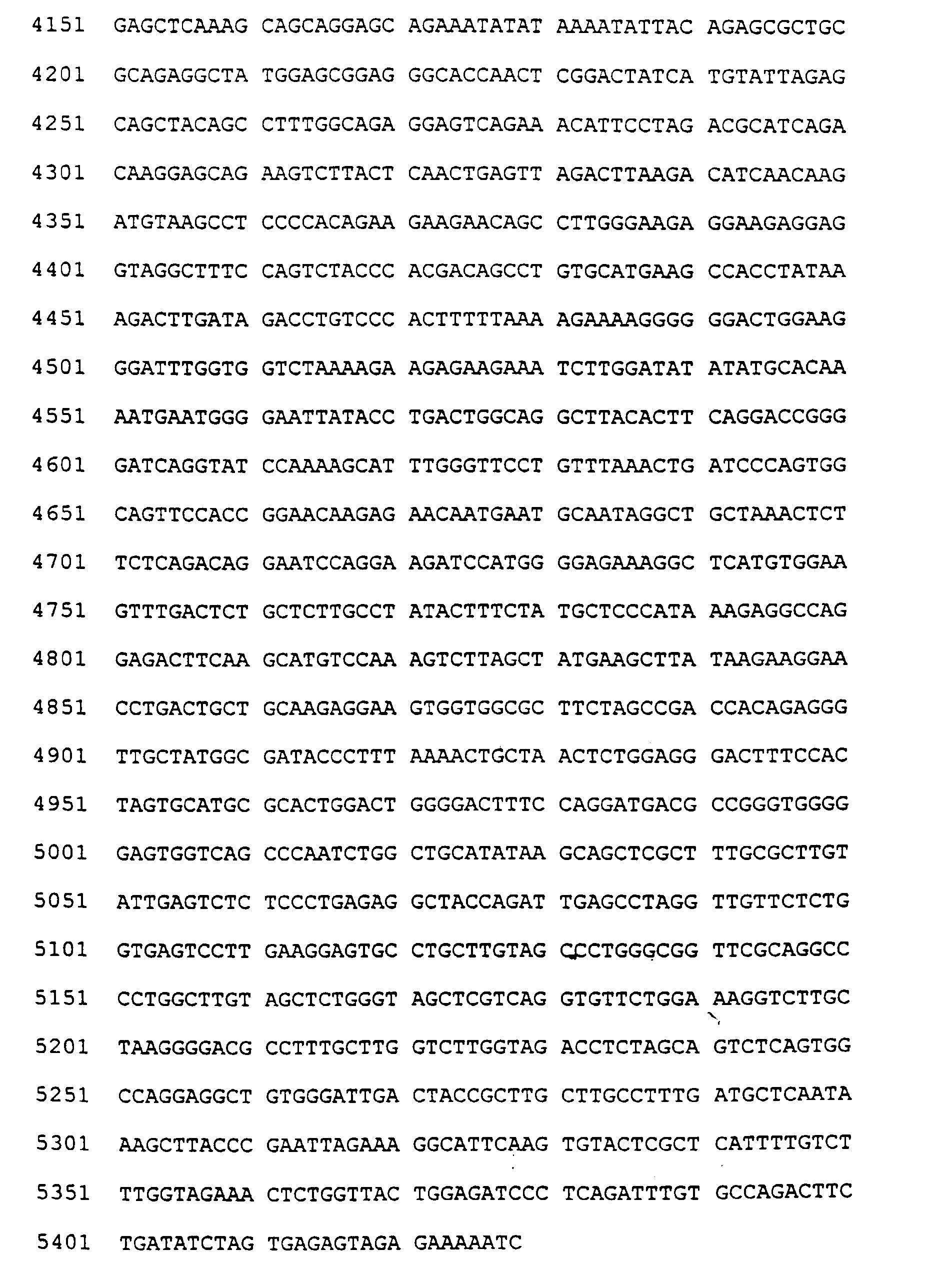

Die Gesamtsequenz, die sich aus der Summe der Sequenzen gemäß Tab. 2 bis 6 ergibt, ist in Tab. 7 dargestellt:

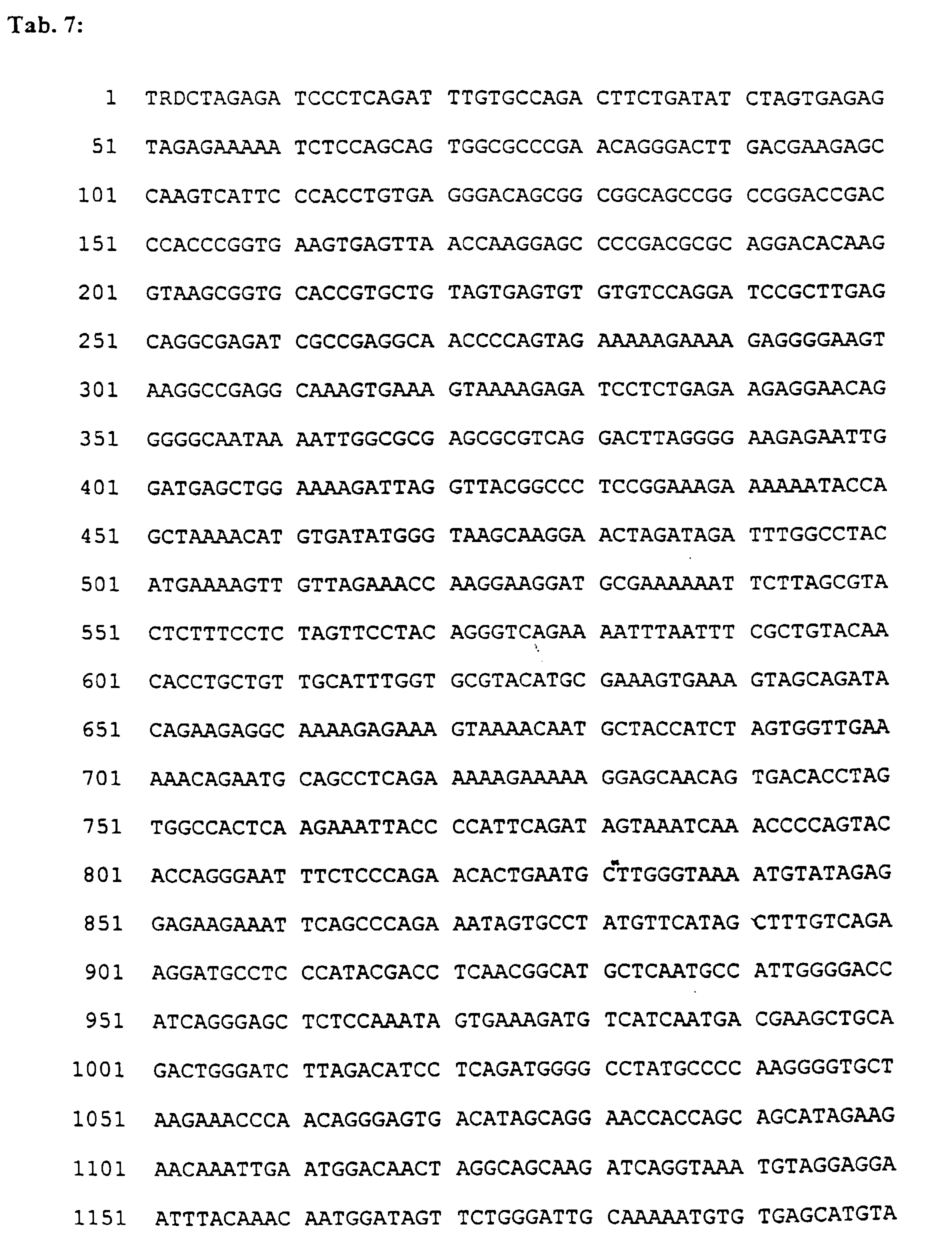

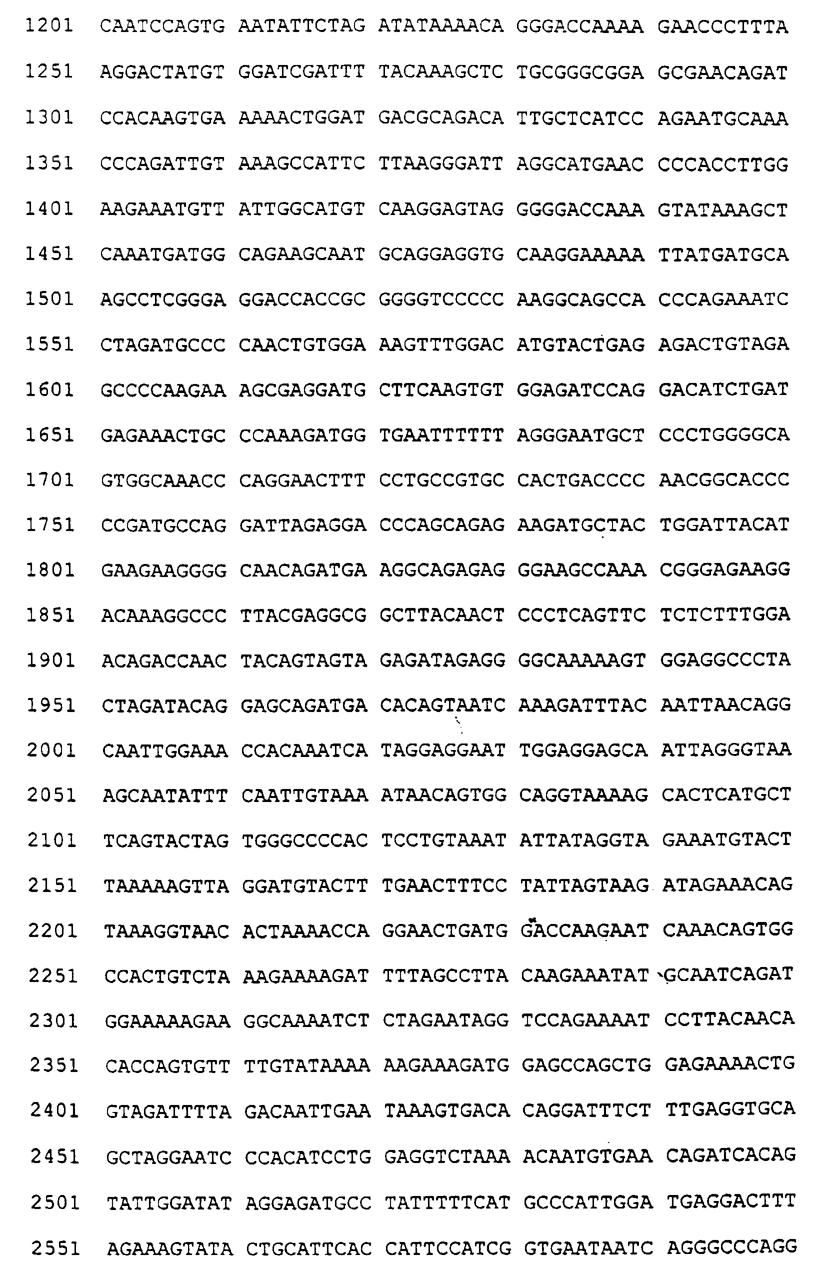

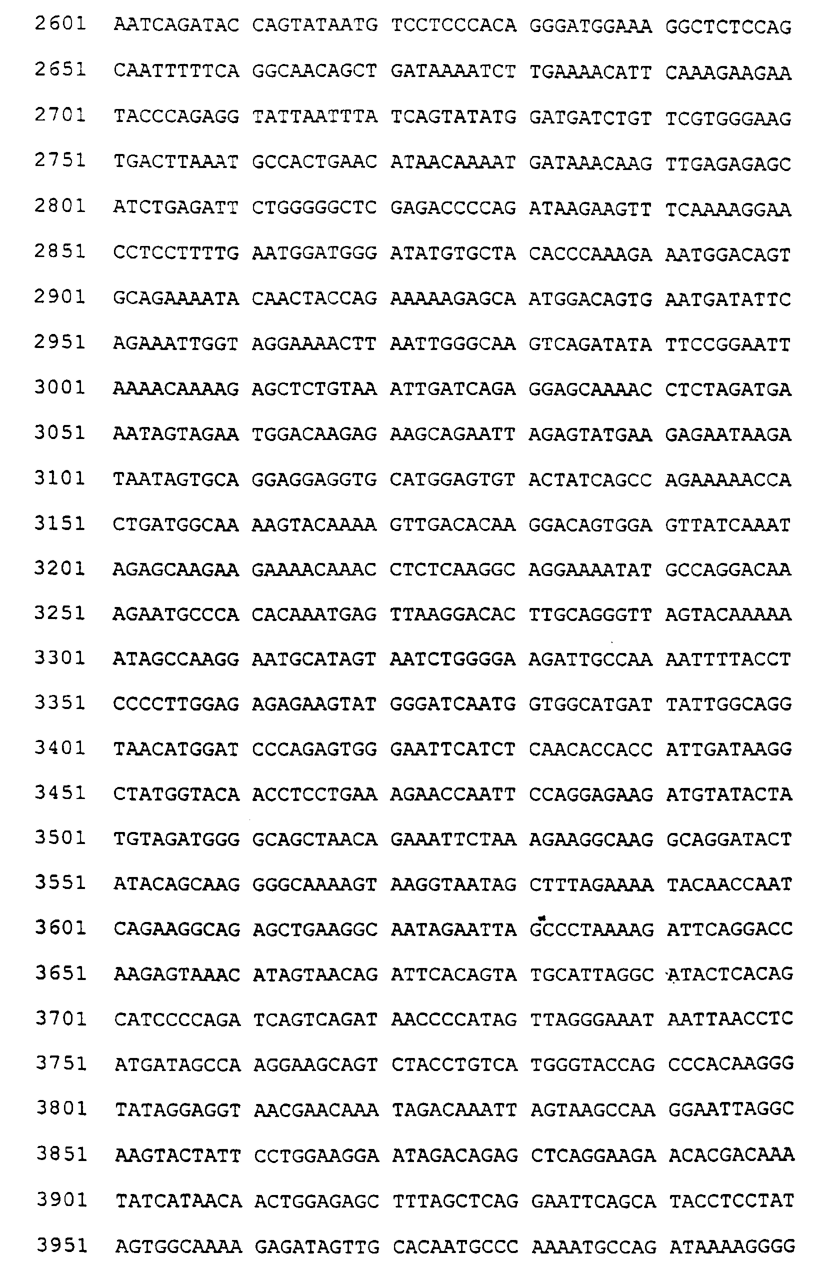

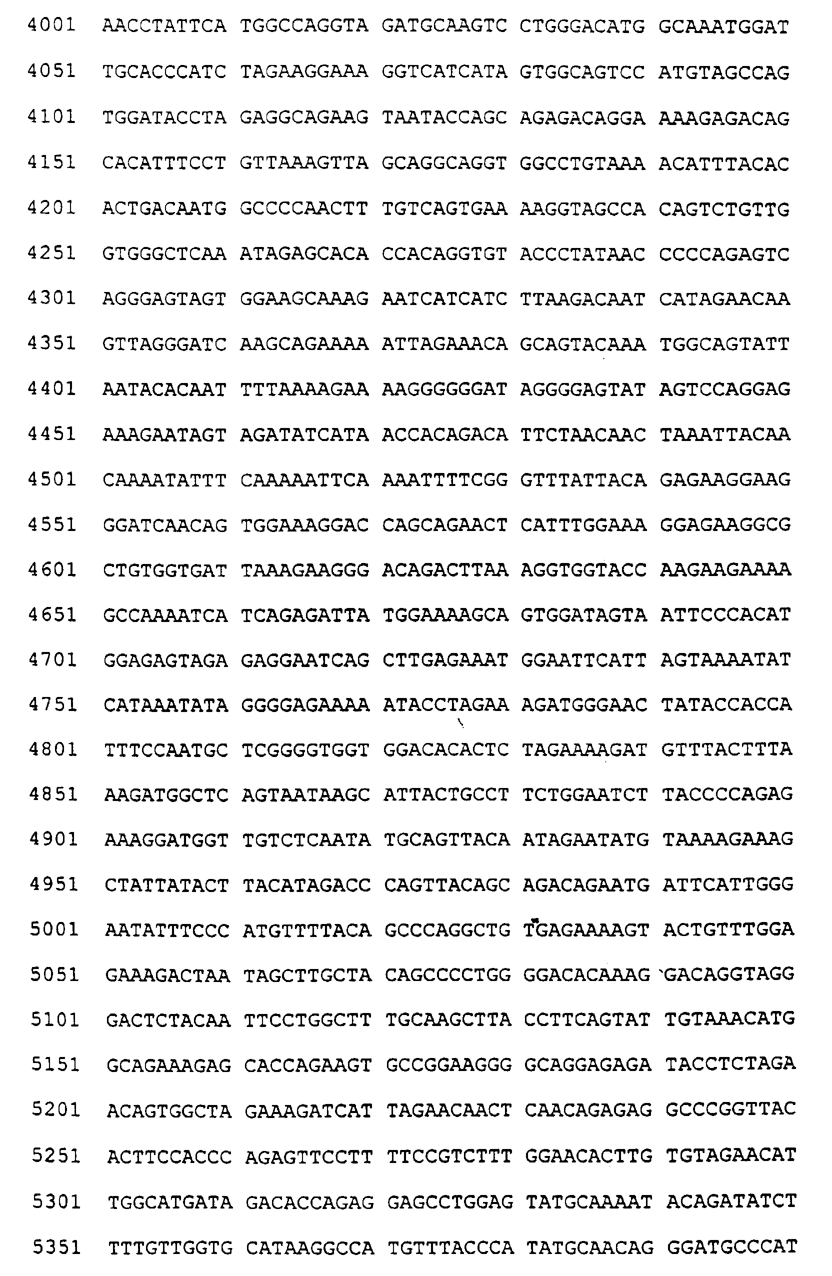

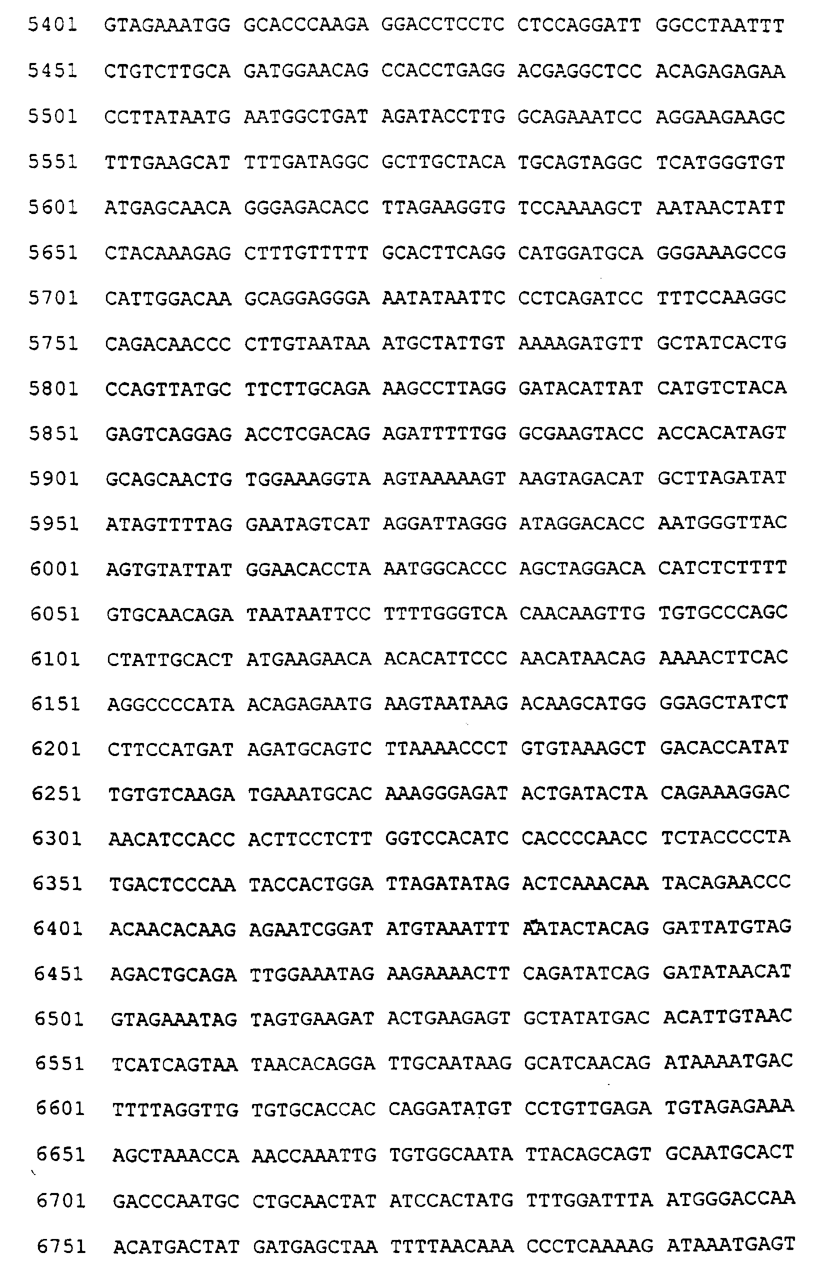

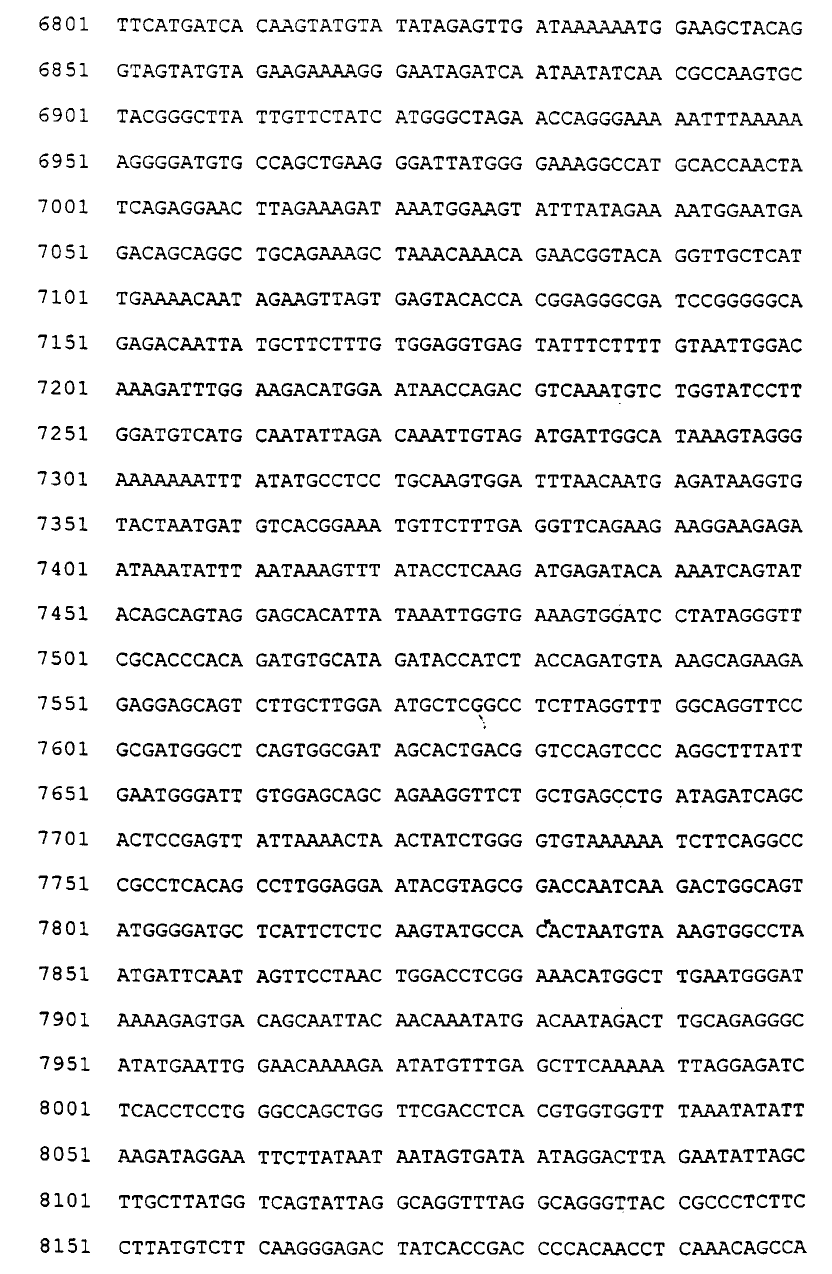

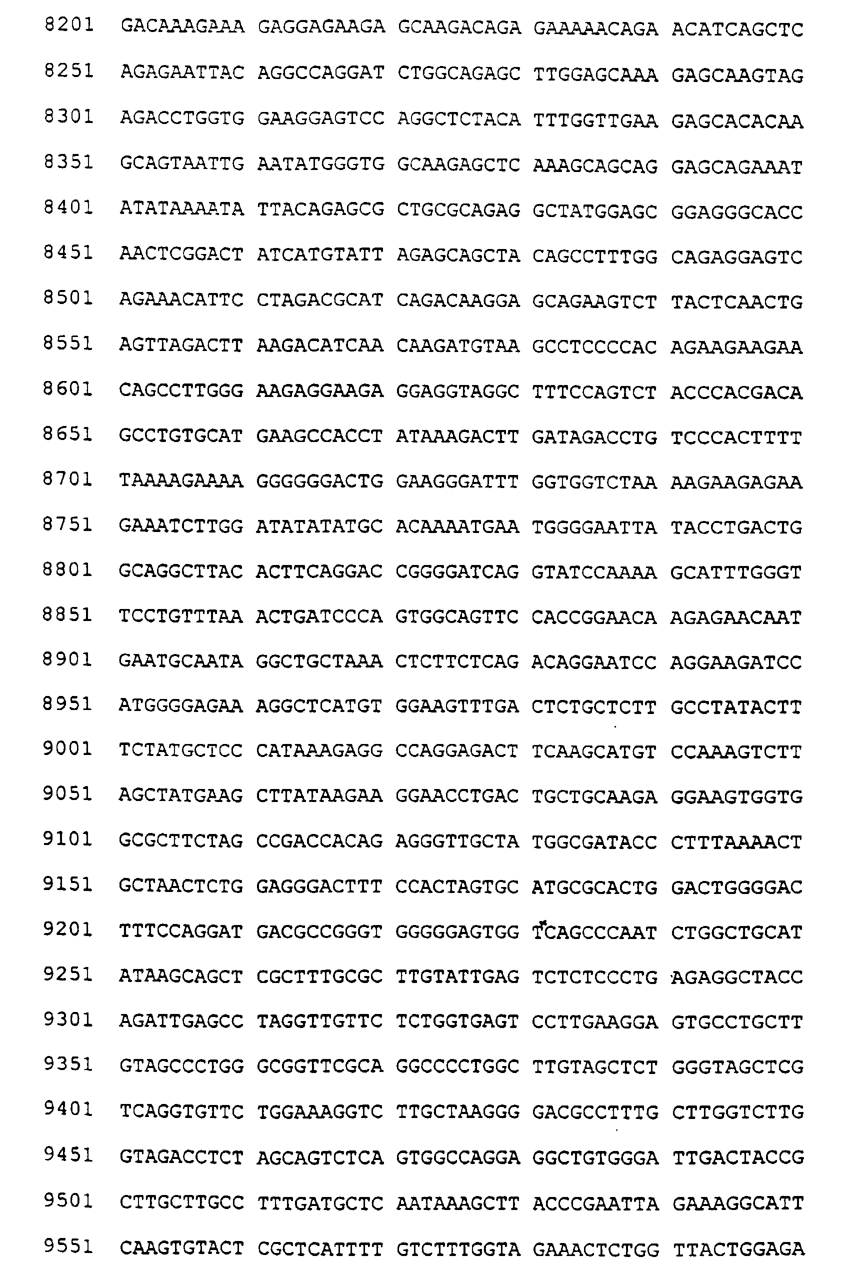

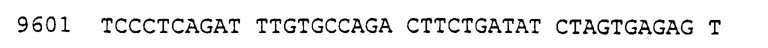

Die Nukleotidsequenz wurde in 3 Leseraster in Aminosäuresequenzen übersetzt, worauf durch Homologievergleiche die Aminosäuresequenzen von GAG (Tab.8), POL (Tab.9) und ENV (Tab.10) identifiziert wurden.

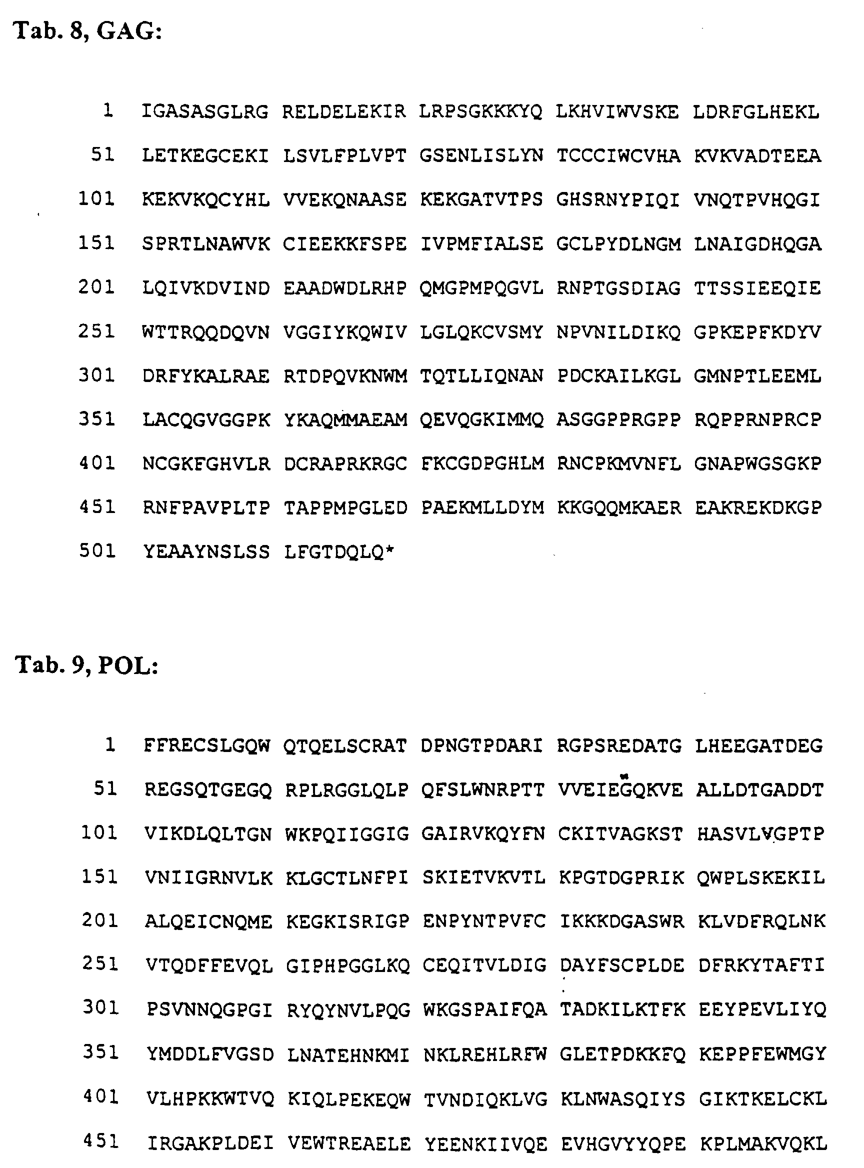

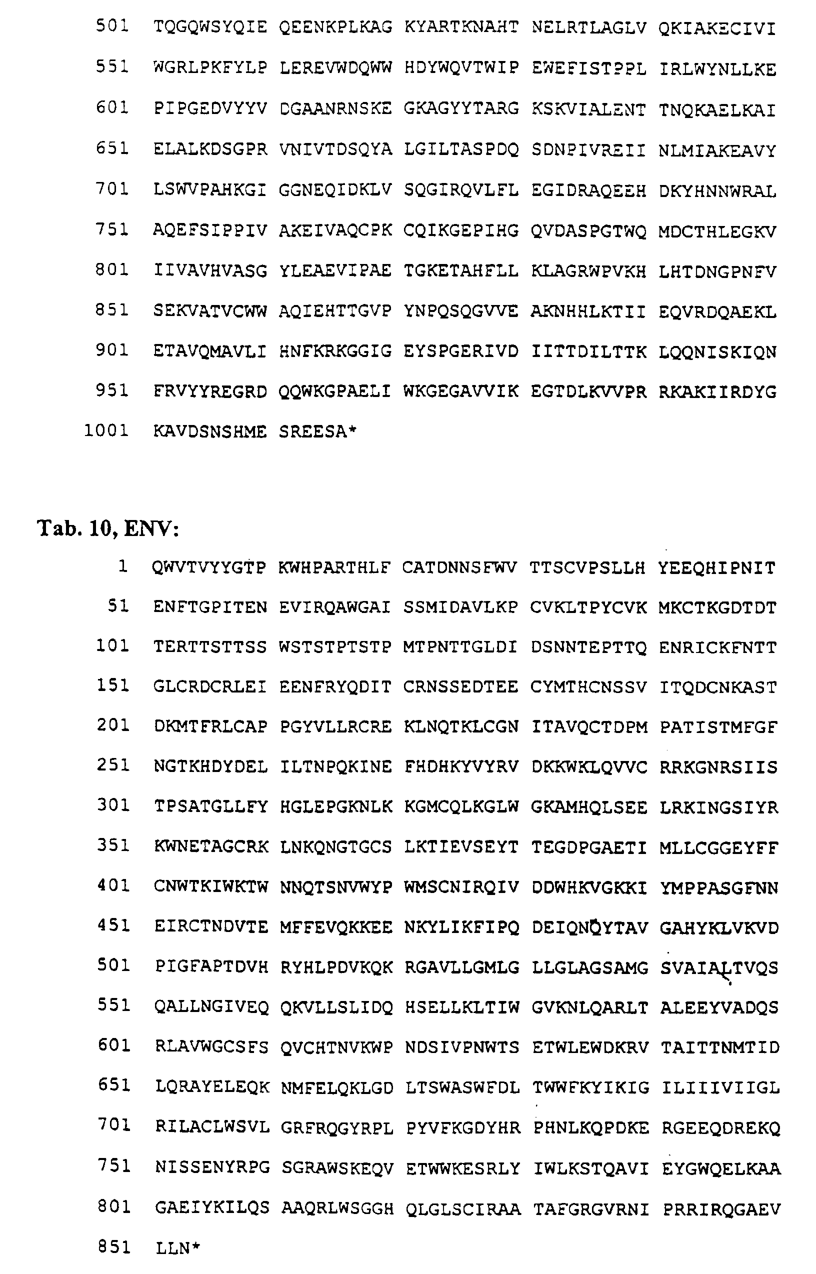

### Beispiel 4:

### Ermittlung der phylogenetischen Stellung von SIM27

### Auswahl der Sequenzen:

Vorn HIV-WWW-Server des LANL (Los Alamos National Laboratory, hiv-web.lanl.gov) wurden 31 HIV- und SIV-Seqenzen ausgewählt, welche alle vollständigen SIV Genome sowie Vertreter der verschiedenen HIV-1- und HIV-2-Subtypen umfaßten. Die folgenden Sequenzen gemäß Tab. 11 wurden berücksichtigt.

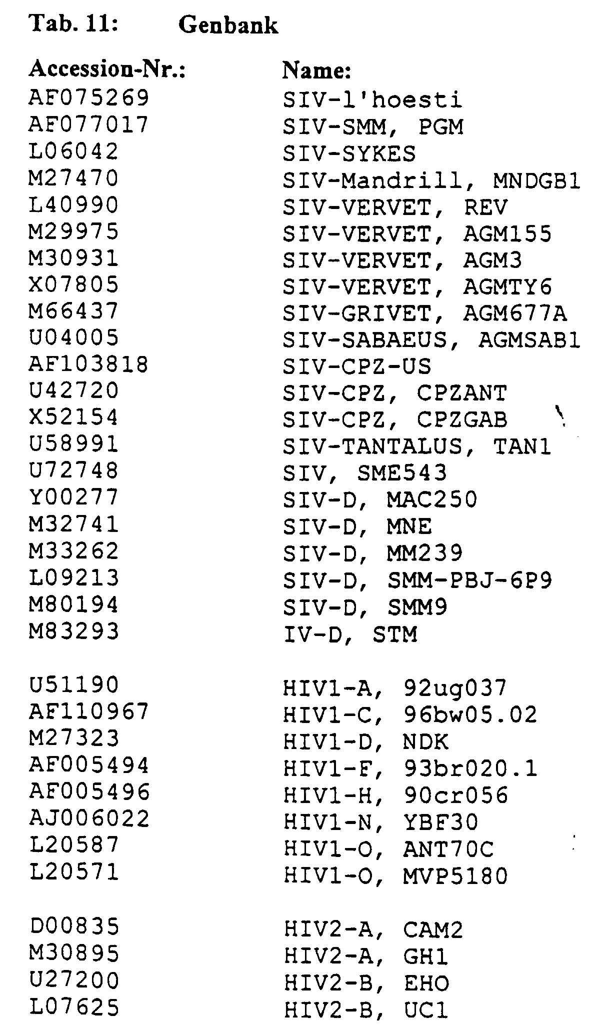

Anhand der Genbank-Accession Numbers dieser Sequenzen wurden die aktuellen Sequenzeinträge aus der Gendatenbank 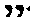 Genbank" extrahiert. Aus diesen Sequenzen wurden anhand der Annotation die Gene env, gag und pol extrahiert und in die Aminosäuresequenz translatiert. Für die Translation wurden nur solche Sequenzen herangezogen, die als funktionell annotiert waren. Pseudogene und nicht als eines der 3 Gene annotierte Genomteile wurden nicht berücksichtigt.

Zusatzlich wurde die Sequenz des Genoms von SIM27 mit der aktuellen Gendatenhank 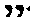 Genbank" verglichen, um keine SIV-Partialsequenz mit hoher Verwandtschaft zu SIM27 zu übersehen. Dabei wurden 2 Partialsequenzen von SIV-rcm (gag und pol) sowie eine pol-Partialsequenz von Mandrillus leucophaeus (Clewley JP et al., J.Virol. 1998; 72: 10305-10309) als zusätzlich relevant identifiziert:
- RCM-GAG: SIV, RCM gag
- RCM-POL: SIV, RCM Pol
- CLEW-POL: SIV, Drill, Clewley

Insgesamt wurden auf diese Weise 4 Datensätze erhalten: 3 Proteindatensätze (env, gag und pol), sowie einer aus genomischen Sequenzen (GENOME).

### Alignment:

Die obigen Sequenzen wurden mit CLUSTALW (Version 1.74) mit Standardeinstellungen zusammen mit den entsprechenden SIM27 Sequenzen aligniert (Thompson J.D et al., Nucleic Acids Res. 22:4673-4680(1994)). Die so erhaltenen Sequenzalignments wurden dann manuell überprüft.

Zu dem pol-Sequenzalignment wurde in jeweils getrennten Analysen noch je einmal die veröffentlichte pol-Teilsequenz vom Drillaffen (Clewley et al.), sowie die pol-Teilsequenz des RCM-Affen dazugefügt. Dasselbe wurde für die GAG-Teilsequenz des RCM-Affen zum gag-Alignment durchgeführt.

Für das Zufügen der Einzelsequenzen zu den Alignments wurde die "Profilealignment Option" von CLUSTALW 1.74 unter Standardeinstellungen benutzt.

So entstanden 3 weitere Proteindatensätze mit kleinen Teilsequenzen RCM-GAG, RCM-POL und DRILL-POL. Jeder dieser Datensätze wurde nur in Bezug auf den Bereich der jeweiligen Teilsequenz betrachtet.

### Phylogenetische Analysen:

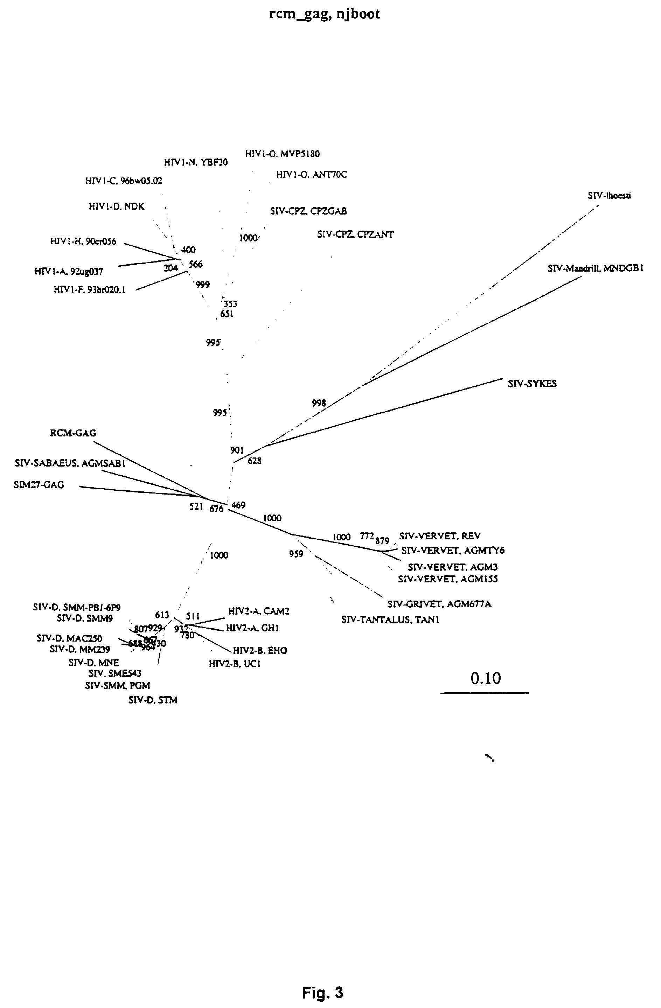

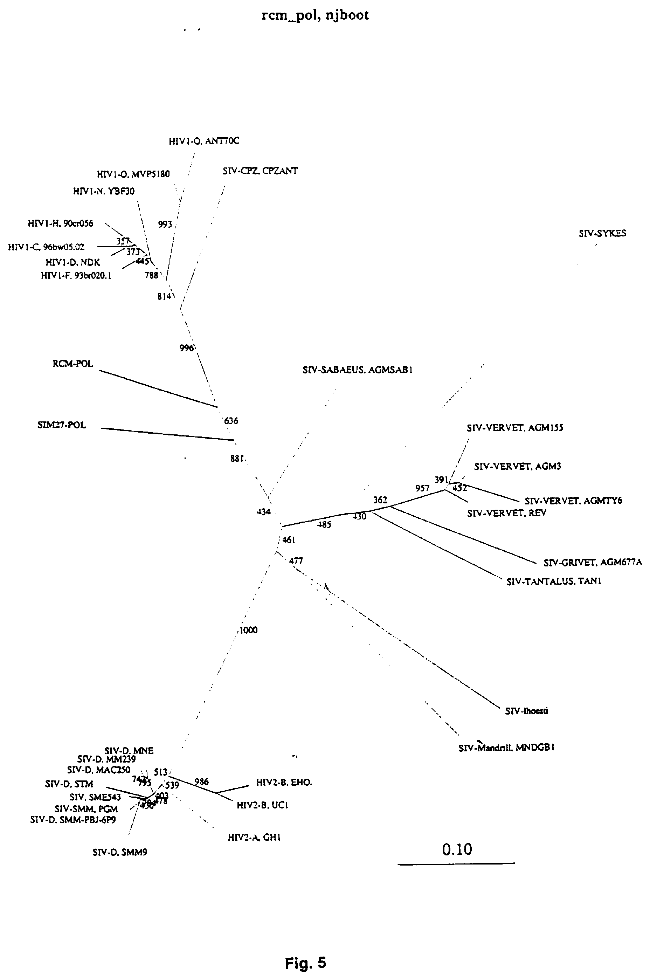

Mit den obigen sieben Alignments (GENOME (Fig.1), GAG (Fig.2), , RCM-GAG (Fig.3), POL (Fig.4), RCM-POL (Fig.5), DRILL-POL (Fig.6), ENV (Fig.7)) wurden nun unabhängig phylogenetische Stammbäume erstellt. Dazu wurde die Neighbor-Joining-Methode, wie sie in CLUSTALW 1.74 implementiert ist, in 1000 Bootstrapanalysen verwendet. Zum Berechnen der Bäume wurden die Standardeinstellungen verwandt, nur wurden alle Alignmentspalten mit Lücken ignoriert, sowie die Korrektur für multiple Mutationen eingeschaltet.

### Beispiel 5:

### Nachweis der diagnostischen Relevanz im Westernblot

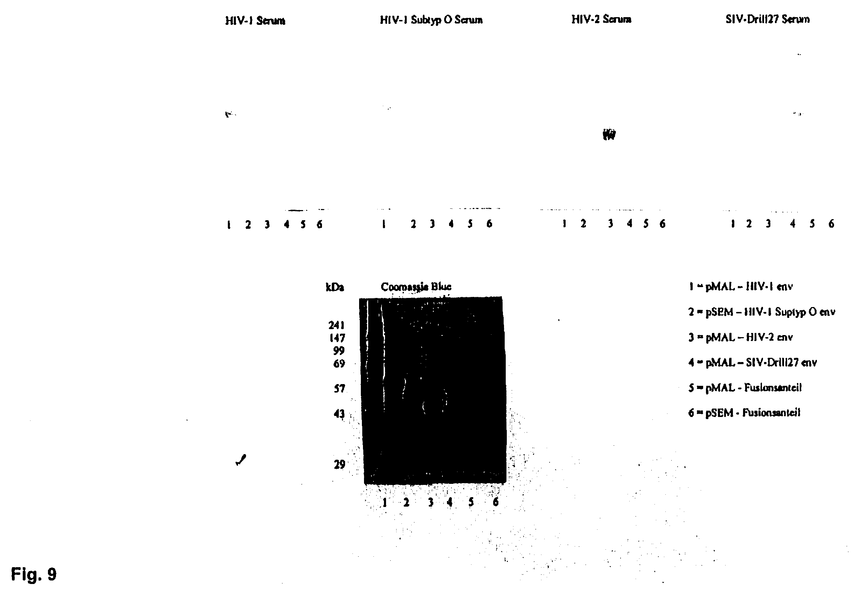

Nach bekannten Methoden der Molekularbiologie (Current Protocols in Molecular Biology, Wiley Interscience, 1994) wurden der den Cystein-Loop enthaltende Bereich von env entweder als Fusion mit dem Maltose bindenden Protein (pMAL-New England Biolabs) oder als Fusion mit β-Gal (Knapp et al., Biotechniques, Vol.8, No.3, 1990) stabil exprimiert. Die Proteine wurden auf Nitrocellulose geblottet, mit den Seren in einer Verdünnung von 1:100 in 5% Magermilch enthaltendern TBS (150mM NaCl, 50 mM Tris pH 8,0) über Nacht inkubiert, mit TBS gewaschen und mit Anti-human-IgG-AP (Sigma A064) und Anti-Affe-IgG-AP (Sigma A1929) für 2 h in einer Verdünnung von 1:1000 inkubiert und mittels Nitrotetrazolium-Blue (Sigma N-6876) und 5-Bromo-4-chloro-indolylphosphat.p-toluidin (Bachem M105) nach Herstellerangaben angefärbt. Es wurden die in Tab. 9 dargestellten Ergebnisse erhalten (Fig.9)

**Tab. 9**

| Protein/Serum | Anti-HIV1 Serum | Anti-HIV1-SubtypO Serum | Anti-HIV2 Serum | Anti-SIV-Drill7-Serum |
|---|---|---|---|---|
| PMAL-HIV1env | +++ | ++ | - | - |
| PSEM-HIV1-SubtypO-env | ++ | ++ | - | - |
| pMAL-HIV2-env | - | - | +++ | - |
| PMAL-SIM27-env | - | - | - | +++ |
| PMAL | - | - | - | - |
| PSEM | - | - | - | - |

Hierbei zeigte sich überraschenderweise, daß der env-Bereich von SIM27 nicht mit Anti-HIV-1, Anti-HIV-1-Subtyp O und Anti-HIV2 Seren reagiert und gleichzeitig Antikörper aus SIM27, welche stark mit SIM27-env reagieren, nicht durch den Einsatz von HIV-1-env, HIV-1-Subtyp O-env und HIV2-env nachgewiesen werden könnten. Daher ist davon auszugehen, daß für den Fall, daß SIM27 oder eine Variante mit vergleichbaren serologischen Eigenschaften den Übergang in die menschliche Population vollziehen sollte, der Nachweis von Antikörpern gegen SIM27 in menschlichen Seren mit den gegenwärtig zum Einsatz kommenden Testen nicht möglich ist, sondern vielmehr SIM27-ENV, oder davon abgeleitete Antigene mit vergleichbaren immunologischen Eigenschaften eingesetzt werden müßten.

### Abbildungen:

### Fig. 1

Phylogenetische Untersuchung der Sequenzen der Tabelle 11 unter Einbeziehung des Gesamtgenoms von SIM27 wie in Beispiel 4 beschrieben durch das Multiple Alignment und die Neighbor-Joining Methode von ClustalW Version 1.74

### Fig. 2

Phylogenetische Untersuchung der aus den Sequenzen der Tabelle 11 extrahierten GAG-Proteine unter Einbeziehung des GAG-Proteins von SIM27 (Tab.8) wie in Beispiel 4 beschrieben durch das Multiple Alignment und die Neighbor-Joining Methode von ClustalW Version 1.74

### Fig. 3

Phylogenetische Untersuchung der aus den Sequenzen der Tabelle 11 extrahierten GAG-Proteine unter Einbeziehung des GAG-Proteins von SIM27 (Tab.8) und der GAG-Partialsequenz von SIVrcm wie in Beispiel 4 beschrieben durch das Multiple Alignment und die Neighbor-Joining Methode von ClustalW Version 1.74

### Fig. 4

Phylogenetische Untersuchung der aus den Sequenzen der Tabelle 11 extrahierten POL-Proteine unter Einbeziehung des POL-Proteins von SIM27 (Tab.9) wie in Beispiel 4 beschrieben durch das Multiple Alignment und die Neighbor-Joining Methode von ClustalW Version 1.74

### Fig. 5

Phylogenetische Untersuchung der aus den Sequenzen der Tabelle 11 extrahierten POL-Proteine unter Einbeziehung des POL-Proteins von SIM27 (Tab.9) und der POL-Partialsequenz von SIVrcm wie in Beispiel 4 beschrieben durch das Multiple Alignment und die Neighbor-Joining Methode von ClustalW Version 1.74

### Fig. 6

Phylogenetische Untersuchung der aus den Sequenzen der Tabelle 11 extrahierten POL-Proteine unter Einbeziehung des POL-Proteins von SIM27 (Tab.9) und der POL-Partialsequenz wie von Clewley (Clewley JP et al., J Virol. 1998; 72: 10305-10309) publiziert und wie in Beispiel 4 beschrieben durch das Multiple Alignment und die Neighbor-Joining Methode von ClustalW Version 1.74

### Fig. 7

Phylogenetische Untersuchung der aus den Sequenzen der Tabelle 11 extrahierten ENV-Proteine unter Einbeziehung des ENV-Proteins von SIM27 (Tab.10) wie in Beispiel 4 beschrieben durch das Multiple Alignment und die Neighbor-Joining Methode von ClustalW Version 1.74

### Fig. 8

Übersicht über die einzelnen PCR-Amplifikationen welche zur vollständigen genomischen Nukleinsäuresequenz von SIM27 führten

### Fig. 9

Westernblot, wie in Beispiel 5 beschrieben.

### Abkürzungen:

- HIV:: Humanes Immunschwächevirus
- SIV:: Simian- (Affen-) Immunschwächevirus
- HTLV:: Humanes T-Lymphoma Virus
- STLV:: Simian-T-Lymphoma Virus
- p:: Protein
- gp:: Glykoprotein
- pol:: Gen der Enzyme von HIV oder SIV, bezeichnet nach der Polymerase
- gag:: Gen der Kernproteine von HIV oder SIV
- env:: Gen der Oberflächen- /Glykoproteine von HIV oder SIV
- IN:: Integrase
- RT:: Reverse Transcriptase
- PR:: Protease

## Patentansprüche

1. Immunschwächevirus SIM27, dessen RNA oder ein Teil davon zu der Sequenz gemäß Tab. 2 oder Tab. 3 oder Tab. 4 oder Tab. 5 oder Tab. 6 oder Tab. 7 komplementär ist.

2. Variante des Immunschwächevirus gemäß Anspruch 1, enthaltend eine RNA, die zu einer DNA komplementär ist, welche nach folgendem Verfahren untersucht wird:
(a) Extraktion der in Tab. 11 genannten Sequenzen aus der Gendatenbank "Genbank" und und Einlesen der Sequenzen einschließlich der zu untersuchenden Sequenz in das Computerprogramm "ClustalW Version 1.74",
(b) multiples Alignment der Sequenzen gemäß Tab. 11 und der zu untersuchenden Sequenz und phylogenetische Analyse der erhaltenen Daten nach der Neighbor-Joining-Methode mittels Computerprogramm 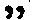 ClustalW Version 1.74",
(c) Visualisierung der erhaltenen Stammbaumdaten mit einem geeigneten Darstellungsprogramm als Stammbaum,
dadurch gekennzeichnet ist, dass die Variante entlang der Strecke vom Endpunkt bis zum ersten Verzweigungspunkt von dem Ast des Stammbaumes abzweigt, an dessen Ende sich SIM27 befindet.

3. Gag-Protein von SIM27 oder eine Variante davon, welche nach folgendem Verfahren untersucht wird:
(a) Extraktion der GAG-Anteile der in Tab. 11 genannten Sequenzen aus der Gendatenbank "Genbank" und Einlesen der korrespondierenden Aminosäuresequenzen in das Computerprogramm "ClustalW Version 1.74",
(b) multiples Alignment dieser Aminosäuresequenzen mit den Sequenz gemäß Tab. 8 und phylogenetische Analyse der erhaltenen Daten nach der Neighbor-Joining-Methode mittels Computerprogramm "ClustalW Version 1.74";
(c) Visualisierung der erhaltenen Stammbaumdaten mit einem geeigneten Darstellungsprogramm als Stammbaum;
dadurch gekennzeichnet ist, dass die Variante entlang der Strecke vom Endpunkt bis zum ersten Verzweigungspunkt von dem Ast des Stammbaumes abzweigt, an dessen Ende sich SIM27-gag befindet.

4. Env-Protein von SIM27 oder eine Variante davon, welche nach folgendem Verfahren untersucht wird:
(a) Extraktion der ENV-Anteile der in Tab. 11 genannten Sequenzen aus der Gendatenbank "Genbank" und Einlesen der korrespondierenden Aminosäuresequenzen in das Computerprogramm "ClustalW Version 1.74",
(b) multiples Alignment dieser Aminosäuresequenzen mit den Sequenzen gemäß Tab.10 und phylogenetische Analyse der erhaltenen Daten nach der Neighbor-Joining-Methode mittels Computerprogramm "ClustalW Version 1.74";
(c) Visualisierung der erhaltenen Stammbaumdaten mit einem geeigneten Darstellungsprogramm als Stammbaum;
dadurch gekennzeichnet ist, dass die Variante entlang der Strecke vom Endpunkt bis zum ersten Verzweigungspunkt von dem Ast des Stammbaumes abzweigt, an dessen Ende sich SIM27-env befindet.

5. Pol-Protein von SIM27 oder eine Variante davon, welche nach folgendem Verfahren untersucht wird:
(a) Extraktion der POL-Anteile der in Tab. 11 genannten Sequenzen aus der Gendatenbank "Genbank" und Einlesen der korrespondierenden Aminosäuresequenzen in das Computerprogramm "ClustalW Version 1.74",
(b) multiples Alignment dieser Aminosäuresequenzen mit den Sequenzen gemäß Tab.9 und phylogenetische Analyse der erhaltenen Daten nach der Neighbor-Joining-Methode mittels Computerprogramm "ClustalW Version 1.74";
(c) Visualisierung der erhaltenen Stammbaumdaten mit einem geeigneten Darstellungsprogramm als Stammbaum;
dadurch gekennzeichnet ist, dass die Variante entlang der Strecke vom Endpunkt bis zum ersten Verzweigungspunkt von dem Ast des Stammbaumes abzweigt, an dessen Ende sich SIM27-pol befindet.

6. Verwendung eines Virus gemäß Anspruch 1 oder eines Proteins gemäß einem oder mehreren der Ansprüche 3 bis 5 zum Nachweis von gegen ein Immundefizienzvirus gerichteten Antikörpern in einer Probe.
